# EUROPÄISCHE PATENTANMELDUNG

(11) **EP 1 214 942 A2**
(43) Veröffentlichungstag der Anmeldung: **19.06.2002**
(21) Anmeldenummer: 02003333.8
(22) Anmeldetag: 17.05.1999
(51) Int. Cl.: A61K 39/00, A61K 39/385, A61P 35/00, A61P 37/04

(54) **Verwendung von mit Bacteriophagen, die ein tumorspezifisches und/oder tumorassoziliertes Antigen auf ihrer Oberfläche tragen, beladenen dendritischen Zellen zur Behandlung von Tumoren**

(30) Priorität: 15.05.1998 DE 19821925; 14.05.1999 WO PCT/EP99/03353
(62) Teilanmeldung aus: 99924991.5
(71) Anmelder: Immunogenec Biotechnologie GmbH, 82152 Planegg/Martinsried (DE)
(72) Erfinder: Die Erfindernennung liegt noch nicht vor
(74) Vertreter: VOSSIUS & PARTNER

(57) **Zusammenfassung**

Die vorliegende Erfindung betrifft eine Zusammensetzung, die einen Phagen oder ein funktionell äquivalentes Fragment davon umfaßt, der/das mindestens ein tumorspezifisches und/oder tumorassoziiertes Antigen als Fusionsprotein mit einem Phagenhüllprotein oder einem funktionell äquivalenten Derivat davon auf seiner Oberfläche exprimiert, und gegebenenfalls einen pharmazeutisch verträglichen Träger und/oder ein pharmazeutisch verträgliches Verdünnungsmittel. Die vorliegende Erfindung betrifft weiterhin ein Verfahren zur Klonierung und Expression von Tumorantigenen auf Phagen, durch die eine spezifische Immunantwort induziert wird. Außerdem betrifft die Erfindung eine durch dieses Verfahren hergestellte Zusammensetzung, die dazu vorgesehen ist, als Injektionspräparat zur spezifischen Immuntherapie von Neoplasien bei Säugetieren und auch beim Menschen, verwendet zu werden. Beispiele derartiger Neoplasien sind Hämoblastosen, vorzugsweise ein malignes Lymphom oder eine Leukämie, ein malignes Melanom, ein urogenitales Karzinom, vorzugsweise ein Nieren-, Blasen-, Prostata- oder Hodenkarzinom, ein gynäkologisches Karzinom, vorzugsweise ein Mamma- Ovarial-, Uterus- oder Zervixkarzinom, ein gastrointestinales Karzinom, vorzugsweise ein Ösophagus-, Magen-, Kolon-, Rektum-, Pankreas- Gallenblasen-, Gallengangs- oder hepatocelluläres Karzinom, ein bösartiger endokrinologischer Tumor, vorzugsweise ein Schilddrüsenkarzinom, ein maligner Lungentumor, vorzugsweise ein Bronchialkarzinom oder Mesotheliom, ein Sarkom oder ein ZNS-Tumor ist.

## Beschreibung

Die vorliegende Erfindung betrifft eine Zusammensetzung, die einen Phagen oder ein funktionell äquivalentes Fragment davon umfaßt, der/das mindestens ein Tumorantigen als Fusionsprotein mit einem Phagenhüllprotein oder einem funktionell äquivalenten Derivat davon auf seiner Oberfläche exprimiert, und gegebenenfalls einen pharmazeutisch verträglichen Träger und/oder ein pharmazeutisch verträgliches Verdünnungsmittel. Die vorliegende Erfindung betrifft weiterhin ein Verfahren zur Klonierung und Expression von Tumorantigenen auf Phagen, durch die eine spezifische Immunantwort induziert wird. Außerdem betrifft die Erfindung eine durch dieses Verfahren hergestellte Zusammensetzung, die dazu vorgesehen ist, als Injektionspräparat zur spezifischen Immuntherapie von Neoplasien bei Säugetieren, insbesondere beim Menschen, verwendet zu werden.

Das Immunsystem ist die körpereigene Abwehr, die sich der Bekämpfung von Krankheitserregern widmet. Es gibt prinzipiell zwei Arten von Abwehrsystemen gegen infektiöse Faktoren: Die spezifische erworbene (adaptive) Immunantwort und die unspezifische angeborene Immunantwort. Im folgenden ist vor allem die adaptive Immunantwort, die erst durch ein Antigen ausgelöst wird, von Bedeutung. Die besonderen Kennzeichen der adaptiven Immunantwort sind Anpassung, Gedächtnis und Spezifizität. Sie sind Folge eines klonalen Selektionsprozesses, der durch Antigenkontakt ausgelöst wird.
Das Immunsystem ist humoral und zellulär organisiert. Im Zuge der humoralen Immunantwort findet unter anderem eine Zerstörung von antikörperbehafteten Tumorzellen durch natürliche Killerzellen (NK-Zellen) statt, die ausgelöst wird, wenn an die Zelloberfläche von Tumoren gebundene Antikörper mit NK-Zellen in Kontakt treten. Diese antikörperabhängige, zellvermittelte Zytotoxizität (ADCC) ist ein sehr effektiver Mechanismus, um Tumorzellen zu zerstören. Siehe hierzu RJ. Dearman et al. 1988 (Blond 72: 1985-91) und George AJ. et al. 1987 (J Immunol.140: 1695-170). Auch im Rahmen der zellulären Immunantwort sieht das Immunsystem eine Bekämpfung von Tumorzellen vor: Eine Aktivierung von T-Lymphozyten setzt jedoch in den meisten Fällen die Wechselwirkung mit antigenpräsentierenden Zellen (APCs) voraus. Hierzu benötigt die T-Zelle ein weiteres kostimulatorisches Signal, welches von den antigenpräsentierenden Zellen geliefert wird. Das Fehlen dieser Kostimuli hat zur Folge, daß die T-Zellen nicht mehr auf eine Aktivierung reagieren. Krebszellen können sich durch das Fehlen von kostimulatorischen Molekülen einer Aktivierung des Immunsystems entziehen.

Einige Tumorzellen können vom Immunsystem erkannt und vernichtet werden. Diese Funktion des Immunsystems wird z.B. deutlich an den gelegentlich beobachteten Spontanremissionen von Tumoren. Bei der Regression von Tumoren spielen unter anderem T-Lymphozyten eine Rolle, wobei die T-Zell-Reaktion gegen einen Tumor die Erkennung von sog. tumorspezifischen Transplantationsantigenen (sog. TSTA) voraussetzt. Unklar ist aber immer noch, warum einige Tumoren eine spontane adaptive Immunantwort auslösen, andere aber nicht. Burnet bezeichnete die Fähigkeit des Immunsystems, Tumorzellen aufzuspüren und zu zerstören als Immunüberwachung bzw. *"immune surveillance*" (Burnet FM. 1970, The concept of immunological surveillance, Prog Exp Tumor Res 13, 1-27). An der weiten Verbreitung von Krebserkrankungen erkennt man jedoch, daß die Immunüberwachung nicht besonders effektiv ist bzw. daß Tumorzellen offensichtlich über immunologische "Escape"-Mechanismen verfügen, die ihnen die Fähigkeit verleihen, sich dem Zugriff des funktionierenden Immunsystems zu entziehen. Tumorzellen können z.B. eine nur gering immunogene Wirkung haben, weil ihnen MHC-Moleküle oder kostimulatorische Moleküle fehlen. Eine weitere Möglichkeit, sich dem Immunsystem zu entziehen, besteht darin, daß Tumorzellen, die vom Immunsystem erkennbare Antigene exprimieren, diese aufgrund einer antikörperinduzierten Aufnahme in die Zelle oder aufgrund von antigener Variation verlieren (Immunselektion). Schließlich produzieren Tumoren oft Substanzen wie z.B. TGF-β, welche die Immunantwort direkt unterdrücken.
Im Laufe der letzten Jahre wurde eine Vielzahl von Tumor-assozierten Antigenen beschrieben, von denen die meisten mittels monoklonaler Antikörper in Mäusen identifiziert wurden (Reisfeld und Sell, Eds.: Monodonal Antibodies and Cancer Therapy, UCLA Symposia on Molecular and Cellular Biology, Vol. 27, Alan R. Liss, 1985, NY, 1-609). Obwohl sich dabei die meisten Antigene als Onkofetal- oder Differenzierungsantigene erwiesen haben und sich ihre Tumorspezifität als quantitatives und nicht als qualitatives Merkmal gezeigt hat, da sie in geringen Maße auch auf normalen Körperzellen vorkommen, sind einige Antigene auch ausreichend spezifisch gegenüber neoplastischen Zellen im Vergleich zu normalen Zellen, um potentiell zur Identifizierung von Tumorzellen und zur Therapie verwendet zu werden. Einige der am besten charakterisierten tumorspezifischen Antigene sind die von neoplastischen Lymphozyten exprimierten Idiotypproteine (Stevenson et al. 1977: Idiotypic determinants on the surface immunoglobulin of neoplastic lymphocytes: a therapeutic target. Fed Proc.; 36:2268-71). Dabei versteht man unter einem Idiotyp die Gesamtheit der antigenen Epitope (Idiotope) in der variablen Region eines Antikörpers. Über diese können Lymphomzellen selektiv vom Immunsystem erkannt werden. Es ist daher nicht verwunderlich, daß bei immuntherapeutischen Strategien in der Lymphomtherapie die Idiotypvakzinierung eine herausragende Stellung einnimmt.
Tierversuche unterschiedlicher Art haben gezeigt, daß eine Immunisierung mit Tumorantigenen zur Abwehr einer anschließenden Gabe von lebensfähigen Krebszellen führen kann. Ein möglicher Ansatz dabei ist die Verwendung von Zellvakzinen, die entweder aus lebenden, abgetöteten oder gentechnisch veränderten Krebszellen bestehen. Die Wirksamkeit der Zellvakzinierung beruht auf der Induktion von zytotoxischen T-Zellen (CTL's), die in der Lage sind, Tumorzellen spezifisch zu zerstören. Bisher scheint es jedoch so zu sein, daß die Effektivität der Zellvakzinierung nicht besonders hoch ist, weil nicht ausreichend viele zytotoxische T-Zellen rekrutiert werden können, um zu einer wirksamen. Tumortherapie zu kommen. Ein anderer Ansatz zur Immuntherapie von Krebserkrankungen ist die Immunisierung mit Tumorantigenen. Die Zerstörung von Tumorzellen wird dabei überwiegend über eine Antikörper-abhängige-zellvermittelte-Zytotoxizität (ADCC) vermittelt. Eines der Hauptprobleme ist dabei die schwache Immunogenität der Tumorantigene. Das liegt daran, daß die tumorassoziierten Antigene, die die Ziele für eine solche Immunantwort darstellen, oft in einigen normalen Zellen, wenn auch in geringen Mengen, vorhanden sind und daher vom Immunsystem als "Selbst" erkannt werden, gegen das im Normalfalle keine Immunantwort stattfindet. Es ist daher nicht sicher, ob Krebspatienten mit diesem Ansatz wirksam behandelt werden können. Im Gegensatz dazu handelt es sich z.B. bei dem Idiotypprotein um ein tumorspezifisches Antigen, das ausschließlich von Lymphomzellen produziert wird. Gegen Idiotypproteine kann, wie in Mäusen und in klinischen Studien gezeigt werden konnte, eine z.T. sehr effektive Immunantwort induziert werden. Die Problematik der Idiotypvakzinierung besteht darin, daß die Idiotypproteine für jeden Patienten individuell hergestellt werden müssen und selbst nur wenig immunogen sind. Idiotypproteine müssen daher an ein immunogenes Trägermolekül, z.B. KLH gekoppelt werden, damit sich eine antiidiotypische Immunantwort induzieren läßt (George AJ et al.: Idiotypic vaccination as a treatment for a B cell lymphoma. J Immunol 1988, 141:2168-74; Levy R. et al.: Therapy of lymphoma directed at idiotypes. J Natl Cancer. Inst Monogr 1990; 1990:61-8). Eine Vakzinierung mit Fragmenten der Idiotypproteine wie z.B. dem CDR3-Peptid ist weit weniger wirksam. (Campbell MJ et al.: Idiotype vaccination against murine B cell lymphoma. Humoral and cellular responses elicited by tumor-derived immunoglobulin M and its molecular subunits. J. Immunol 1987; 139: 2825-33). Es zeigte sich darüberhinaus, daß eine korrekte Faltung der Idiotypproteine ganz entscheidend für die Wirksamkeit der Immuntherapie ist, die im wesentlichen über antiidiotypische Antikörper vermittelt wird. Bis vor kurzem mußten Idiotypproteine über aufwendige Hybridomtechniken hergestellt werden. Heute ist es jedoch möglich, eine patientenspezifische Idiotypvakzine gentechnisch z.B. in Form eines Einzelstrang-Fragments herzustellen (Hawkins RE et al.: Idiotypic vaccination against human B-cell lymphoma. Rescue of variable region gene sequences from biopsy material for assembly as single-chain Fv personal vaccines. Blood 1994; 83:3279-88), Diese Einzelstrang-Fragmente wurden mittlerweile als DNA-Vakzine erfolgreich getestet (Spellerberg MB et al.: NA vaccines against lymphoma: promotion of anti-idiotypic antibody responses induced by single chain Fv genes by fusion to tetanus toxin fragment C., J Immunol 1997; 159:1885-92 und King CA et al.: DNA vaccines with single-chain Fv fused to fragment C of tetanus toxin induce protective immunity against lymphoma and myeloma., Nat Med 1998; 4:1281-6). Single chain (sc) Idiotypvakzine ließen sich auch in Tabakpflanzen herstellen und erfolgreich zur Vakzinierung verwenden. (McCormick AA et al.: Rapid production of specific vaccines for lymphoma by expression of the tumor-derived single-chain Fv epitopes in tobacco plants. Proc Natl Acad Sci USA 1999; 96:703-8). Im Zuge der zunehmenden Bedeutung der Tumore hinsichtlich ihrer absoluten Häufigkeit, die hauptsächlich auf demographische Gründe zurückzuführen ist, werden viele Anstrengungen unternommen, um den Krebs durch geeignete Maßnahmen wirksam, aber gleichzeitig auch schonend für den Patienten zu behandeln.

Phagen sind virusähnliche Partikel, die Bakterien infizieren und sich in ihnen vermehren können.
λ-Bakteriophagen werden gegenwärtig häufig zur Erstellung von cDNA-Bibliotheken eingesetzt. Zu diesem Zweck haben sich λ-ZAP®-Vektoren bewährt, wie sie in der US-A 5,128,256 (Huse, Sorge, Short) beschrieben sind. In dieser Patentschrift ist ein Verfahren offenbart, bei dem Gene mit der hohen Effizienz des Phagensystems kloniert werden können und durch den Schritt der in vivo-Exzision rekombinante Phagemid-Vektoren (z.B. BlueScript®-Vektoren) ohne weitere Subklonierungsprozesse hergestellt werden können.
Filamentöse Phagen sind längliche 7 x 900 nm große langgestreckte Phagenpartikel. Sie können gramnegative Bakterien infizieren und sich hier vermehren. Sie bestehen aus einem länglichen Kapsid in helikaler Anordnung, das die einzelsträngige Virus-DNA umgibt. Die Virushülle setzt sich aus fünf Phagenproteinen zusammen, die aus dem Major Coat Protein cpVIII und den vier Minor Coat Proteinen cplll, cpVI, cpVII und cplX bestehen. Weil die Coat-Proteine cplll und cpVIII in den periplasmatischen Raum von gramnegativen Bakterien sezerniert werden, um hier als Bestandteil der Phagenoberfläche zusammengesetzt zu werden, sind sie ideale Zielmoleküle für die Fusion mit Idiotypen (siehe hierzu US-A 5,658,727 (Barbas, Kang, Lerner), erteilt am 19. Aug. 1997). Filamentöse Phagen finden gegenwärtig im Rahmen der "Phage Display"-Technologie breite Anwendung. Unter "Phage Display"-Technologie versteht man die Expression von Peptiden oder Proteinen in Fusion mit Phagen-Hüllproteinen auf der Phagenoberfläche. Besonders eignen sich dabei die Hüllproteine III und VIII. Das 50 Aminosäure große gVIII Protein ist eines der Hauptbestandteile der Phagenhülle und liegt in ca. 2700 Kopien in der Hülle vor: Das gIII-Protein ist mit seinen 406 Aminosäuren wesentlich größer, kommt jedoch in nur 3-5 Kopien am infektiösen Ende des Phagen vor. Das glll Protein spielt bei der Infektion von Bakterien eine große Rolle. Weitere Coat-Proteine, die etwa ähnlich häufig wie das gIII-Protein auf der Phagenhülle vorkommen sind das gVI-, gVII- und gIX-Protein, wobei das gVI-Protein der Verankerung des gIII-Proteins dient und die gVII- und glX-Proteine am anderen Ende des Phagen lokalisiert sind.
Die "Phage Display" Technologie findet gegenwärtig vor allem bei der Identifizierung von Peptidliganden, im Epitop Mapping und bei der Selektion von rekombinanten Antikörpern breite Anwendung.
Die Technologie kann aber auch verwendet werden, um Immunogene zu produzieren. So kann gegen auf Phagen exprimierte antigene Epitope sowohl mit als auch ohne Adjuvantien eine z.T. gute (protektive) Immunantwort induziert werden. Siehe Arbeiten von de la Cruz et al.1988 (JBC 263,4318-22), Greenwood et al. 1991 (JMB 220:821-27), Minenkova et al. 1993 (Gene 128:85-88), Meola et al. (J. Immunol. 154, 3162-72), Bastien et al. 1997 (Virol. 234,118-22). Des weiteren beschreibt DE 69124800 T2 gentechnologisch veränderte filamentöse Phagen, die unter anderem immunogene Oligopeptide von FMDV oder des Malariaerregers Plasmodium falciparum auf ihrer Oberfläche exprimieren. Den dort beschriebenen Immunogenen ist gemeinsam, daß von Phagen exprimierte kleine antigene Oligopeptide zur Vakzinierung verwendet werden. Dabei werden die Peptide entweder in Fusion mit dem gIII oder gVIII-Protein exprimiert. Durch Immunisierung mit solchen Phagen kann mit und ohne Zusatz eines Adjuvants eine spezifische T-Zell-abhängige humorale Immunantwort gegen die antigenen Peptide induziert werden, die spezifisch mit den ursprünglichen Antigenen kreuzreagieren (siehe Willis et al., Gene 1993; 128, 97-83), wobei eine immunogene Wirkung bevorzugt bei "gVIII-Vakzinen" nachzuweisen war, zumindest aber "gVIII-Vakzine" wirksamer waren als "gIII-Vakzine", möglicherweise weil sie eine höhere Antigendichte auf ihrer Oberfläche aufweisen. In der Literatur findet sich bislang kein Hinweis, daß von Phagen exprimierte größere Proteine (bzw. Polypeptide) als Immunogene wirksam sind. Vielmehr würde ein Fachmann sogar davon abraten, komplexe Proteine auf Phagen zu exprimieren, da dadurch die Beladung mit Antigen um ein Vielfaches erhöht würde und damit die Induktion einer Immunantwort entweder überhaupt nicht mehr möglich oder nicht spezifisch genug ist. Durch die Expression größerer Proteine oder Domänen auf Phagen konnte in Unkenntnis der Befunde der vorliegenden Erfindung darüberhinaus erwartet werden, daß das biologische Verhalten in ungewünschter Form verändert werden kann, wodurch die Verwendung von Phagenvakzinen unsicher wird.

In der Patentschrift DE 3703702 A1 wird ein Impfstoff gegen Melanome beschrieben, der im wesentlichen reine antigene Peptide oder Proteine, die zu dem Melanomassoziierten p97-Antigen gehören, umfaßt. Die Peptide oder Proteine können auch von rekombinanten Viren, wozu auch Bakteriophagen gerechnet werden, exprimiert werden, wodurch Vakzinformulierungen auf Basis inaktivierter Viren oder auf Basis von Lebendviren hergestellt werden können. DE 3703702 A1 enthält allerdings keinerlei Lehre zu einem möglichen Herstellungsverfahren von rekombinanten Bakteriophagen, die derartige zu p97 gehörende Peptide oder Proteine umfassen. Rekombinante Phagen herzustellen, bei denen immunogene Proteine auf der Oberfläche exprimiert werden, ist jedoch aus sterischen Gründen nicht möglich, sondern nur dann, wenn Phagenhybride hergestellt werden. Es besteht deshalb lediglich die Möglichkeit einer Konjugation an ein Trägermolekül, das Immunogenität verleiht. Entsprechend wird das allgemeine Konzept, zu p97 gehörende antigene Strukturen als Fusionsprotein mit einem Hüllprotein von Viren oder Bakteriophagen zu präsentieren, nicht erwähnt.

Eine spezifische Immuntherapie gegen einen Tumor herbeizuführen, setzt die Kenntnis eines Tumorantigens voraus. Bei Kenntnis eines solchen Antigens kann die Immuntherapie künstlich durch Zufuhr eines spezifischen Antikörpers herbeigeführt werden oder durch Immunisierung mit einem Tumorantigen. Die Therapie mit monoklonalen Antikörpern hat jedoch den Nachteil, daß die biologischen Halbwertszeit im Körper nur kurz ist und deshalb lang anhaltende Anti-Tumoreffekte nicht beobachtet werden. Darüber hinaus ist das Risiko von Tumor-Escape-Mechanismen sehr hoch, weil sich die Immunantwort nur gegen ein Epitop richtet. Der Vorteil der aktiven Immuntherapie durch Immunisierung mit einem Tumorantigen liegt hingegen in der lang anhaltetenden Immunantwort. Am Beispiel der Idiotypvakzinierung zeigen sich besonders deutlich die Vorteile einer solchen aktiven Immunisierung: Induktion einer polyklonalen, lymphomspezifischen und langanhaltenden Immunantwort. Klinisch konnte sich die Idiotypvakzinierung jedoch bis heute nicht durchsetzen, weil Idiotypvakzine für jeden Patienten individuell hergestellt werden müssen, was bisher nur mit aufwendigen, zeitraubenden und kostspieligen Hybridomtechniken möglich ist. Die Herstellung von Idiotypvakzinen als lösliche sc-Fragmente beinhaltet demgegenüber abgesehen von der Faltungsproblematik die Schwierigkeit, diese in größeren Mengen zu produzieren und sie für den therapeutischen Einsatz aufzureinigen. Es kann auch nicht erwartet werden, daß die exprimierten Tumorantigene notwendigerweise Immunogene sind. Das der vorliegenden Erfindung zugrunde liegende technische Problem war dementsprechend, ein Verfahren zu bereitzustellen, welches eine kostengünstige und schnelle Herstellung von patientspezifischen Tumorvakzinen ermöglicht. Vorzugsweise sollten diese auch gute Immunogene sein.

Dieses technische Problem wird durch die Bereitstellung der in den Ansprüchen charakterisierten Ausführungsformen gelöst.

Somit betrifft die vorliegende Erfindung eine Zusammensetzung, die einen Phagen oder ein funktionell äquivalentes Fragment davon umfaßt, der/das mindestens ein tumorspezifisches und/oder tumorassoziiertes Antigen als Fusionsprotein mit einem Phagenhüllprotein oder einem Fragment oder einem funktionell äquivalenten Derivat davon auf seiner Oberfläche exprimiert, und gegebenenfalls einen pharmazeutisch verträglichen Träger und/oder ein pharmazeutisch verträgliches Verdünnungsmittel.
Der Begriff "funktionell äquivalentes Fragment" eines Phagen umfaßt im Sinne dieser Erfindung Fragmente von Phagenpartikel, die durch physikalische, chemische und/oder biologische Behandlung hergestellt wurden und sich aufgrund dieser Behandlung(en) morphologisch und/oder strukturell und/oder funktionell von nativen Phagenpartikeln unterscheiden, jedoch im wesentlichen die gleichen immunologischen, vorzugsweise immunstimulatorischen Eigenschaften wie native Phagenpartikel aufweisen. Ein "funktionell äquivalentes Fragment" eines Phagen umfaßt beispielsweise Phagenpartikel, deren Genom durch chemische Behandlung zerstört wurde ohne das Nucleokapsid und/oder die Hülle zu verändern, oder Phagenpartikel, deren Genom und/oder Nucleokapsid und/oder Hülle durch z.B. enzymatische, Hitze- und/oder Ultraschall-Behandlung verändert wurde.

Der Begriff "funktionell äquivalentes Derivat" eines Phagen-Hüllproteins umfaßt im Sinne der vorliegenden Erfindung ein Phagen-Hüllprotein, das eine N- und/oder C-terminale und/oder interne Deletion aufweist, und/oder das zusätzlich zu der natürlich vorkommenden Aminosäuresequenz eine Insertion aufweist oder/und dessen Aminosäuresequenz sich von der natürlich vorkommenden Aminosäuresequenz durch eine oder mehrere Substitutionen unterscheidet, wobei zumindest die Fähigkeit zur Interaktion mit anderen Molekülen (z.B. dem viralen Genom oder anderen viralen Hüllproteinen), die für das Virion-"Assembly" notwendig ist, erhalten ist. Geeignete "Fragmente" sind dem Fachmann bekannt.

Der Begriff "tumorspezifisch" bzw. "tumorassoziiert" bezeichnet im Sinne der vorliegenden Erfindung Antigene, die Bestandteile von Krebszellen sind und aus dem Zytoplasma, der Zelloberfläche oder dem Kern entstammen können. Vorzugsweise werden diese Antigene auf der Tumorzelloberfläche als Oberflächenantigene exprimiert, sie können jedoch auch intrazellulär vermehrt nachweisbar sein. Eine Präsentation auf einer Tumorzelle kann dann z.B. auch im Kontext von MHC Klasse II-Molekülen erfolgen.
"Tumorspezifische Antigene" sind erfindungsgemäß Polypeptide, die ausschließlich von Tumorzellen gebildet werden. Das können tumorspezifische Neoantigene sein, die z.B. durch Genumlagerung, Mutationen oder allgemein als Folge einer Transformation in maligne Zellen entstanden sind. Ein typisches Beispiel für ein tumorspezifisches Antigen ist das von Lymphomzellen exprimierte Idiotypprotein. Weiterhin können tumorspezifische Antigene auch Proteine sein, die durch Aktivierung von unterdrückten Genen entstanden, wenn sie nur in oder auf Tumorzellen vorkommen. "Tumorassoziierte Antigene" sind hingegen definitionsgemäß Antigene, die zwar bevorzugt in oder auf Tumorzellen vorkommen, aber auch auf nicht-malignen Zellen nachweisbar sind. Beispielsweise können sie als physiologisch vorkommende Differenzierungsantigene oder als Zwischenprodukte der Ontogenese eines Zelltyps nachweisbar sein, die mit weiterer Differenzierung aus normalen Zellen wieder verschwinden. Manche dieser Antigene lassen sich auch bei bestimmten krankhaften Prozessen in Nicht-Tumorzellen vermehrt nachweisen. Beispiele solcher Antigene sind onkofetale Antigene wie das carcinoembryonale Antigen (CEA) bei Kolonkarzinomen, das "squamous cell carcinoma"-Antigen (SSC) bei epithelialen Tumoren, α₁-Fetoprotein bei prim. Leberzell-Carzinom, Isoferritin und fetales Sulfoglykoprotein bei Magen- und Kolonkarzinom, α₂-H-Ferroprotein bei frühkindlichen Malignomen oder γ-Fetoprotein bei Sarkomen, Leukämien und Mamma-Carzinom Andere erfindungsgemäße Beispiele sind das Tennessee-Antigen (Tennagen), "tissue-polypeptide"-Antigen TPA, onkofetale Membran-Antigene (OFMA), tumorspezifische Transplantations-Antigene (TSTA), membran-assoziierte Antigene (MATA) sowie Kryptantigene wie z.B. das "A-like" Antigen.
"Tumorassoziierte Antigene" im Sinne der vorliegenden Erfindung sind ferner Polypeptide, die in geringer Kopienzahl auf normalen, nicht-tumorigenen Zellen und in hoher Kopienzahl von Tumorzellen produziert werden, so daß in diesem Fall die Spezifität für die Tumorzelle nicht qualitativer, sondern quantitativer Natur ist.

Vorzugsweise werden tumorassoziierte Antigene in einer Kopienzahl präsentiert, die eine quantitatives Bestimmung z.B. über durchflußzytometrische Verfahren ermöglicht, wobei die Signalintensität ("Mean Fluorescence"-Wert) gleich oder höher liegt als für normale Zellen bevorzugt, jedoch mindestens um den Faktor 10 bzw. eine log-Stufe höher ist als bei normalen Zellen, was sich an einem deutlichen Rechts-Shift der Kurve im Einzelparameter-Histogramm zeigt. Ein Beispiel eines solchen Antigens ist das Onkogen Bcl-2, welche z.B. in Lymphomzellen deutlich überexprimiert ist. Tumorassoziierte Antigene im Sinne der vorliegenden Erfindung sind ferner Antigene, die eine effektive Tumortherapie ermöglichen, auch wenn diese nicht spezifisch ist.
Der Begriff "tumorspezifisches Antigen" bzw. "tumorassoziiertes Antigen" umfaßt im Sinne der vorliegenden Erfindung auch Fragmente der oben erwähnten Oberflächenantigene, die im wesentlichen der jeweiligen extrazellulären Domäne entsprechen. Vorzugsweise weisen die Oberflächenantigene bzw. deren Fragmente eine Länge von mindestens 20 Aminosäuren auf, vorzugsweise 30, 40 oder 50 stärker bevorzugt eine so große Anzahl von Aminosäuren, daß die Länge mindestens eine immunogene Domäne des Tumorantigens in ihrer dreidimensionalen Struktur umfaßt und am meisten bevorzugt die. vollständige primäre Aminosäuresequenz eines nativen Tumorantigens. Des weiteren umfassen die o.g. Begriffe im Sinne der vorliegenden Erfindung Oberflächenantigene sowie deren Fragmente, deren Aminosäuresequenz durch eine oder mehrere Substitutionen von der natürlich vorkommenden Aminosäuresequenz abweicht, wobei die Aminosäure-Substitutionen vorzugsweise konservative Austausche darstellen. Solche Austausche umfassen beispielsweise den Austausch einer neutralen, hydrophoben, den Austausch einer neutralen, polaren, den Austausch einer basischen oder den Austausch einer sauren Aminosäure durch eine Aminosäure der jeweiligen selben Klasse, die dem Fachmann bekannt sind.

Die erfindungsgemäße Zusammensetzung ist vorzugsweise ein Arzneimittel.
In einer besonders bevorzugten Ausführungsform ist das Arzneimittel ein Vakzin.

Wie oben erwähnt, kann die Zusammensetzung der Erfindung einen pharmazeutisch verträglichen Träger und/oder ein pharmazeutisch verträgliches Verdünnungsmittel umfassen. Beispiele geeigneter Träger sind dem Fachmann bekannt und umfassen z.B. Phosphat-gepufferte Salzlösungen, Wasser, Emulsionen wie z.B. Öl/Wasser-Emulsionen, verschiedene Arten von Netzmittel oder Detergenzien, sterile Lösungen, etc. Zusammensetzungen, die solche Träger umfassen, können mittels bekannten konventionellen Verfahren hergestellt werden.
Die erfindungsgemäßen Zusammensetzungen können einem Individuum in geeigneter Dosierung verabreicht werden. Eine Verabreichung kann oral oder parenteral erfolgen, z.B. auf intravenösem, intraperitonealem, subkutanem, perinodalem, intramuskulärem, topischem, intradermalem, intranasalem, oder intrabronchialem Wege oder über einen Katheter an einer Stelle in einer Arterie. Die Höhe der Dosierung wird von dem behandelnden Arzt bestimmt und hängt im wesentlichen von den klinischen Faktoren ab. Diese Faktoren sind in der Medizin und der Wissenschaft bekannt und umfassen beispielsweise die Körpergröße bzw. das Gewicht, die Körperoberfläche, das Alter, das Geschlecht, und den allgemeinen Gesundheitszustand des Patienten, die spezielle zu verabreichende Zusammensetzung, die Behandlungsdauer, die Art der Verabreichung und die eventuelle gleichzeitige Behandlung mit anderen Arzneimittel. Eine typische Dosis kann z.B. in einem Bereich zwischen 0,001 und 1000 µg liegen, wobei Dosen unterhalb oder oberhalb dieses beispielhaften Bereiches, vor allem unter Berücksichtigung der oben erwähnten Faktoren, vorstellbar sind. Im allgemeinen sollte sich bei regelmäßiger Verabreichung der erfindungsgemäßen Zusammensetzung die Dosis in einem Bereich zwischen 1 µg- und 10 mg-Einheiten pro Tag befinden. Wird die Zusammensetzung intravenös verabreicht, was nicht bevorzugt empfohlen wird, um die Gefahr einer anaphylaktischen Reaktionen zu minimieren, sollte sich die Dosis in einem Bereich zwischen 1 µg- und 10 mg-Einheiten pro Kilogramm Körpergewicht pro Minute befinden.
Die Zusammensetzung der Erfindung kann lokal oder systemisch verabreicht werden. Präparate für eine parenterale Verabreichung umfassen sterile wäßrige oder nicht-wäßrige Lösungen, Suspensionen und Emulsionen. Beispiele für nicht-wäßrige Lösungsmittel sind Propylenglykol, Polyethylenglykol, pflanzliche Öle wie z.B. Olivenöl, und organische Esterverbindungen wie z.B. Ethyloleat, die für Injektionen geeignet sind. Wäßrige Träger umfassen Wasser, alkoholisch-wäßrige Lösungen, Emulsionen, Suspensionen, Salzlösungen und gepufferte Medien. Parenterale Träger umfassen Natriumchlorid-Lösungen, Ringer-Dextrose, Dextrose und Natriumchlorid, Ringer-Laktat und gebundene Öle. Intravenöse Träger umfassen z.B. Flüssigkeits-, Nährstoff- und Elektrolyt-Ergänzungsmittel (wie z.B. solche, die auf Ringer-Dextrose basieren). Die erfindungsgemäße Zusammensetzung kann außerdem Konservierungsmittel und andere Zusätze umfassen, wie z.B. antimikrobielle Verbindungen, Antioxidantien, Komplexbildner und inerte Gase. Desweiteren können, abhängig von der beabsichtigten Verwendung, Verbindungen wie z.B. Interleukine, Wachstumsfaktoren, Differenzierungsfaktoren, Interferone, chemotaktische Proteine oder ein unspezifisches immunmodulatorisches Agens enthalten sein.
Durch die erfindungsgemäße Zusammensetzung wird entgegen den Erwartungen eine tumorspezifische Immunantwort ausgelöst, welche eine protektive Immunität bei Vertebraten gegenüber einer Gabe von Tumorzellen ausübt und im wesentlichen auf einer humoralen aber auch auf einer zellulären Immunantwort basiert. In vitro konnte gezeigt werden, daß dendritische Zellen, die mit den erfindungsgemäßen Phagenvakzinen gepulst wurden, eine T-Zellproliferation induzieren. Es konnte außerdem in Zytotoxizitäsassays nachgewiesen werden, daß eine spezifische Lyse der Lymphomzellen durch Bcl-2 exprimierende Phagen auftritt. Die als Kontrollen mitgeführten Daudi-Idiotyp Phagen konnten hingegen wie erwartet keine zytotoxische Immunantwort induzieren, da Daudi Lymphomzellen aufgrund des ß2-Mikroglobulin Defekts keine Histokompatibilitätsantigene exprimieren und somit für eine zytotoxische T-Zell-Antwort nicht empfänglich sind. Diese Daten legen nahe, daß über antigenpräsentierende Zellen in vitro eine spezifische T-Zell abhängige Immunantwort ausgelöst werden kann. Diese Daten legen außerdem nahe, daß diese Eigenschaften auch in vivo eine große Rolle spielen und somit neben der erwarteten humoralen Immunantwort auch eine spezifische zellvermittelte Anti-Tumorantwort induziert wird, wodurch der Effekt der Phagenvakzine potentiell verstärkt wird. Eines der wesentlichen Merkmale dürfte dabei die bevorzugte Aufnahme der Phagen durch antigenpräsentierende Zellen sein, wodurch eine effektive Beladung mit Antigen stattfindet. Entgegen den allgemeinen Erwartungen erwies sich die Beladung mit Antigen dabei offensichtlich als nicht zu hoch.
Die erfindungsgemäße Impfzusammensetzung erlaubt eine schnelle und kostengünstige Produktion von tumorspezifischen Phagenvakzinen, wobei die Kenntnis von immunogenen Epitopen vorteilhafterweise nicht bekannt sein muß. Ebensowenig müssen die Liganden der Tumorantigene näher charakterisiert sein, noch müssen die exprimierten Tumorantigene dieselben funktionellen Effekte aufweisen. Ein weiterer Vorteil besteht darin, daß durch die Verwendung von komplexen Proteinen eine Immunisierung gegen mehrere Epitope eines Tumorantigens erfolgt, was eine polyklonale gegen den Tumor gerichtete Immunantwort induziert und somit das Risiko von Tumor-Escape-Mechanismen senkt. Dieser Effekt kann im Sinne dieser Erfindung durch die Expression mehrerer unterschiedlicher Tumorantigene auf Phagen im Sinne einer polyvalenten Vakzine noch weiter verstärkt werden. Ein weiterer Vorteil ist, daß durch die erfindungsgemäße Zusammensetzung Epitope nicht nur in ihrer linearen Form, wie etwa bei der Verwendung von Oligo-Peptiden, sondern auch in ihrer dreidimensionalen strukturabhängigen Form erkannt werden können. Diese Eigenschaften sind besonders vorteilhaft, da sie die Voraussetzung z.B. für wirksame Idiotypvakzinierung sind. Bei den Idiotypvakzinen zeigt sich weiterhin, daß sich die Präsentation der Idiotypproteine auf Phagen sehr vorteilhaft auf die Immunisierung auswirkt, da die Imunantwort gegen die nur schwach immunogenen Idiotypproteine durch die Phagen verstärkt wird und damit erst voll nutzbar wird.
Die erfindungsgemäße Injektionszusammensetzung kann prinzipiell bei allen Säugetieren und insbesondere auch bei Menschen eingesetzt werden, um zur Bekämpfung von Neoplasien eine polyklonale, gegen den Tumor gerichtete antikörpervermittelte Immunantwort zu induzieren. Vorteile dieser Präparate sind ein langanhaltender Schutz des Individuums. Die Zusammensetzung hat, wie oben bereits erwähnt, z.B. die Form einer sterilen Lösung, Emulsion oder Suspension. Sie kann gegebenenfalls eine geeignete antimikrobielle Substanz, Hilfsmittel zur Isotonisierung sowie zur Isohydrie bzw. Euhydrie beeinhalten. Weiterhin können Stoffe zur chemischen Stabilisierung (z.B. Antioxidantien) und Substanzen zur unspezifischen Verstärkung der Immunantwort, wie z.B. das Aluminiumhydroxid, zugesetzt werden. Die Zusätze sollen die immunisierende Wirkung verbessern, ohne dabei die Verträglichkeit negativ zu beeinflußen.
In einer bevorzugten Ausführungsform ist die erfindungsgemäße Zusammensetzung ein Injektionspräparat zur spezifischen Immuntherapie von Neoplasien bei Säugetieren, besonders bevorzugt beim Menschen.

In einer weiteren bevorzugten Ausführungsform ist die erfindungsgemäße Zusammensetzung ein Injektionspräparat zur patientenspezifischen Behandlung von Lymphomen, insbesondere Non-Hodgkin-Lymphomen, oder ein Injektionspräparat zur spezifischen Immuntherapie von malignen Melanomen, urogenitalen, gynäkologischen und/oder gastrointestinalen Karzinomen, Schilddrüsenkarzinomen und/oder Bronchialkarzinomen.

In einer am meisten bevorzugten Ausführungsform ist die erfindungsgemäße Zusammensetzung für die subkutanen, intradermalen, perinodalen oder intraperitonealen Applikationen/Verabreichungen konfektioniert.

In einer anderen bevorzugten Ausführungsform der erfindungsgemäßen Zusammensetzung exprimiert der Phage oder das funktionell äquivalente Fragment davon mindestens ein Cytokin auf seiner Oberfläche. Dabei ist das mindestens eine Cytokin, vorteilhafterweise ein Interleukin, vozugsweise IL-1α, IL-1ß, IL-2, IL-4, IL-6, IL-10, IL-12, IL-13, II-15 und/oder IL-18, ein Wachstums- oder Differenzierungsfaktor, vorzugsweise GM-CSF, G-CSF, M-CSF und/oder Flt3-Ligand, ein Mitglied der TNF-Familie, vorzugsweise TNF-α, LT-α LT-β,, OX-40, 4-1BB oder CD40-Ligand, ein chemotaktisches Protein, vorzugsweise MCP-1, ein Interferon, vorzugsweise IFN-α und/oder IFN-γ, oder ein anderes immunmodularorisches Agens. Cytokine können auch funktionelle ähnlich wirkende virale Proteine sein oder Fragmente oder analoge Proteine mit derselben funktionellen Wirkung wie das Cytokin.

Das mindestens eine Cytokin wird dabei vorzugsweise als Fusionsprotein mit einem Phagen-Hüllprotein oder einem funktionell äquivalenten Derivat davon exprimiert.

In einer anderen bevorzugten Ausführungsform der Zusammensetzung der vorliegenden Erfindung gehört der Phage zur Familie der Corticoviridae, Cystoviridae, Inoviridae, Leviviridae, Lipothrixviridae, Microviridae, Myoviridae, Plasmaviridae, Podoviridae, Siphoviridae, Sulpholobus shibatae Viren (SSV) oder Tectiviridae.

In einer besonders bevorzugten Ausführungsform ist der Phage ein Lambda-, PM2-, ⁻6-Cystovirus-, Acholeplasma-, MS2-, Qbeta, Thermoproteus1, φX174-, Spiroplasma-, Mac-1-, T1-, T2-, T3-, T4-, T5-, T6-, T7-, P1-, µ2-, MS2-, M20- oder S13-Phage.

In einer weiteren bevorzugten Ausführungsform ist der Phage ein filamentöser Phage.

In einer alternativen Ausführungsform wird bei der Herstellung von rekombinanten ZAP®-Phagen das Tumorantigen(TA)-Fusionsprotein in den periplasmatischen Raum der Bakterien transportiert, wo es strukturell seine native Faltung erhält und dann bei der Lyse der Bakterien ins Medium abgegeben wird, weil λ-Bakteriophagen nicht die für filamentöse Phagen typische cpIII-Proteine auf der Zelloberfläche tragen. Mit anderen Worten werden bei diesem Verfahrensstand tumorspezifische Antigene als lösliche Fusionsproteine exprimiert, die beispielsweise über eine Affinitätssäule aufgereinigt werden können. Zur Erleichterung dieser Prozedur kann das tumorspezifische Antigen mit einer Erkennungssequenz versehen sein. In einem weiteren Schritt kann nach in vivo-Exzision das mit cplll fusionierte TA auf der Oberfläche der filamentösen Phagen verankert werden. Die Gene der TA werden entweder in Fusion mit cplll und/oder cpVIII exprimiert. Vom cpIII-Protein existieren auf der Phagenoberfläche 3-5 Kopien. TA in Fusion mit diesem Molekül können in ein bis zwei Kopien vorliegen. Das Hüllprotein VIII kommt auf Phagen wesentlich häufiger vor (2700-3000 Kopien), so daß mit cpVIII fusionierte TA in multiplen Kopien, vorzugsweise bis zu 100 Kopien auf der Phagenoberfäche vorliegen können.

In einer besonders bevorzugten Ausführungsform der erfindungsgemäßen Zusammensetzung ist der filamentöse Phage ein Klasse I-Phage, vorzugsweise ein fd-, M13-, f1-, If1-, Ike-, ZJ/Z- und Ff-Phage ist oder ein Klasse II-Phage, vorzugsweise ein Xf-, Pf1- und Pf3-Phage ist.

In einer zusätzlichen besonders bevorzugten Ausführungsform ist das Phagen-Hüllprotein cpIII und/oder cpVIII oder ein funktionell äquivalentes Fragment davon. In der vorliegenden Beschreibung bzw. in den vorliegenden Ansprüchen werden die Begriffe "cpIII" und "gIII" bzw. "cpVIII" und "gVIII" synonym verwendet.
In einer am meisten bevorzugten Ausführungsform der Zusammensetzung der vorliegenden Erfindung wird das mindestens eine tumorspezifische und/oder tumorassoziierte Antigen als Fusionsprotein mit cpVIII oder einem funktionell äquivalenten Fragment davon und das mindestens eine Cytokin als Fusionsprotein mit cplll oder einem funktionell äquivalenten Fragment davon exprimiert.
In einer weiteren bevorzugten Ausführungsform ist das mindestens eine tumorspezifische und/oder tumorassoziierte Antigen ein Einzelstrangfragment, eines lymphomspezifischem Idiotypproteins oder eine (oder mehrere) antigene Determinante(n) des Idiotypproteins. Das Einzelstrangfragment ist vorzugsweise ein scFv-Fragment oder ein (chimäres) single-chain Fab-Fragment. Letzteres kann z.B. menschliche V_{H} und V_{L}-Ketten und einen Maus-C-Teil aufweisen. In einer bevorzugten Ausführungsform werden die umgelagerten Gene der variablen Fragmente der Immunglobuline amplifiziert und über ein Linkerpeptid zu einem Einzelstrang verknüft, siehe Hawkins, et al. 1994. *(Blood.* 83:3279-88). Diese werden dann in Fusion mit einem Hüllprotein gIII oder gVIII der Phagen, vorzugsweise VIII, exprimiert. Diese Ausführungsform ermöglicht die Expression von richtig gefalteten strukturell unversehrten Idiotypproteinen auf der Oberfläche von Phagen.

In einer anderen Ausführungsform betrifft die vorliegende Erfindung ein Verfahren zur Herstellung der erfindungsgemäßen Zusammensetzung, wobei das Verfahren folgende Schritte umfaßt:
(a) Züchtung einer Wirtszelle, die mit einem wie oben charakterisierten Phagen infiziert wurde, unter Bedingungen, die die Herstellung dieses Phagens erlauben;
(b) Isolierung dieses Phagens aus der Kultur; und gegebenenfalls
(c) Kombinierung des in Schritt (b) isolierten Phagens mit einem pharmazeutisch verträglichen Träger und/oder Verdünnungsmittel.

Die Isolierung des Phagens erfolgt aus dem Bakterienüberstand. Die Konzentrierung erfolgt im allgemeinen durch PEG-/NaCl-Fällungen. Durch mehrmalige Fällungen können Phagen von bakteriellen Kontaminationen sehr effektiv befreit werden. Eine weitere Aufreingung, mit dem Ziel die Phagenpräparation frei von Endotoxinen zu machen, kann über mehrere Verfahren erfolgen, wobei prinzipiell die Möglichkeit der Aufreinigung über CsCI-Dichtegradientenzentrifugation, Uitrazentrifugation, Polymyxin B-Säulenchromatographie oder der TritonX-114 Zwei-Phasenseparation und die Kombination der verschiedenen Verfahren besteht. Als besonders vorteilhaft hat sich bei uns die Triton X-114 Zwei-Phasenseparation erwiesen, mit der bei mehrmaliger serieller Anwendung eine Reduktion der Endotoxinkontamination von initial >300.000 EU/ml auf unter 10EU/ml möglich ist, siehe Aida, Y. et al. 1990 (*J Immunol Methods.* 132:191-5). Diese Separation stellt ein Merkmal einer bevorzugten Ausführungsform des erfindungsgemäßen Verfahrens dar.

In einer anderen oder bevorzugten Ausführungsform betrifft die Erfindung ein Verfahren zur Herstellung der erfindungsgemäßen Zusammensetzung, die zur Induktion einer tumorspezifischen Immunantwort bei Vertebraten geeignet ist, wobei das Verfahren folgende Schritte umfaßt:
(a) Gewinnung von Nucleinsäuren aus einer individuellen Tumorzelle, wobei aus den Nucleinsäuren die Gene von tumorspezifischen Antigenen amplifiziert werden;
(b) Klonierung, vorzugsweise nach gelelektrophoretischen Aufreinigungsverfahren und Verdauung mit geeigneten Restriktionsenzymen, der Gene in ein Vektorsystem; und
(c) Expression der Gene in einem Phagen-Expressionssystem.
Das Herstellungsverfahren nach der vorliegenden Erfindung ermöglicht durch die gezielte Abfolge der oben erwähnten Verfahrensschritte in einer kurzen Zeitspanne eine Klonierung und Expression von Tumorantigenen und Antigenrezeptoren auf Phagen. Eine nach diesem Verfahren hergestellte Injektionszusammensetzung ruft, wie bereits erwähnt, eine polyklonale, gegen den Tumor gerichtete, antikörpervermittelte Immunantwort hervor.

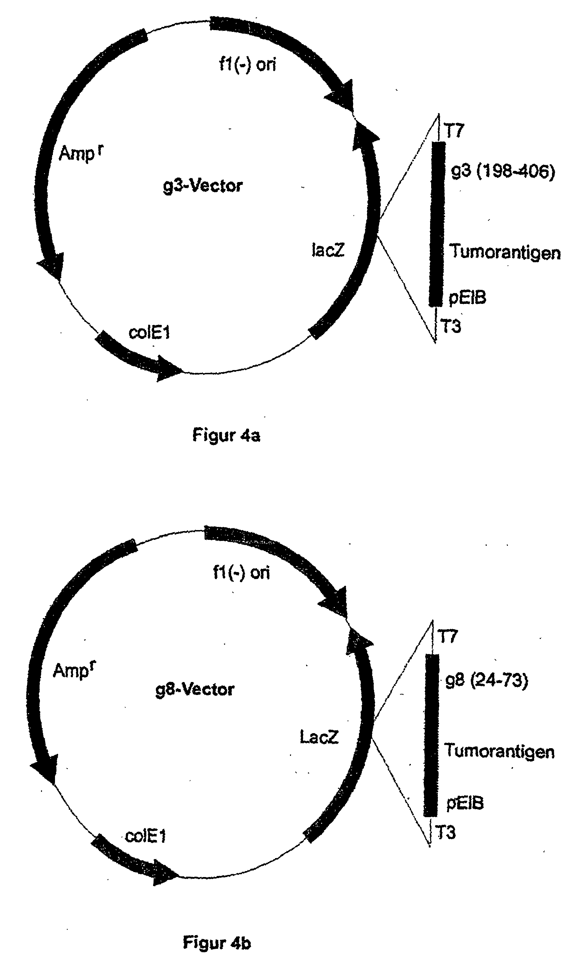

Die Schritte des Verfahrens der vorliegenden Erfindung zur Herstellung einer Injektionszusammensetzung werden im folgenden gemäß einer bevorzugten Ausführungsform detailliert erläutert:
Die individuellen Tumorzellen werden aus dem Tumorkonglomerat nach herkömmlichen Verfahren wie sie z.B. Sambrook et al., s. u. beschrieben werden, gewonnen.
Die Nucleinsäure, vorzugsweise RNA, wird isoliert und in cDNA umgeschrieben und kann dann einer RT-PCR-Methode zur schnellen Amplifikation unterworfen werden, um die Gene der Tumorantigene oder Fragmente daraus zu vermehren.
Durch z.B. ein gelelektrophoretisches Aufreinigungsverfahren (Agarose-Gelelektrophorese) werden die DNA-Fragmente entsprechend ihrer Größe aufgetrennt und aufgereinigt. Durch eine Anschluß-PCR-Reaktion werden Erkennungssequenzen für Restriktionsendonukleasen in die PCR-Produkte eingefügt, um eine nachfolgende Klonierung zu ermöglichen. Nach Aufreinigung des Gens eines tumorspezifischen Antigens beispielsweise mit Geneclean® (Bio 101 Inc., La Jolla, CA, USA) werden die Gene mit unterschiedlichen Restriktionsendonukleasen gespalten. Einer Aufreinigung und Konzentrierung der DNA-Fragmente folgt dann die Ligation dieser DNA-Fragmente in einen Phagemidvektor. Bei diesem Phagemidvektor handelt es sich um einen Vektor wie er z.B. in Fig. 4 skizziert ist. In diesen Phagemidvektor wird das Gen vorzugsweise N-terminal im Leserahmen mit dem Gen eines Hüllproteins kloniert. Vorzugsweise handelt es sich bei den Hüllproteinen um gIII oder gVIII. In einer bevorzugten Ausführungsform befindet sich ein kurzer Gly-Ser-Linker zwischen den Proteinen. Im Gegensatz zu dem allgemein üblichen Phagedisplay-Vektoren werden in dieser erfindungsgemäßen und weiteren bevorzugten Ausführungsform(en) bevorzugt Vektoren verwendet, die keine supprimierbare Amber-Mutation oder deren Äquivalent vor dem Hüllprotein tragen, da das auf die Präsentationsdichte einen negativen Einfluß haben könnte. Ein Erkennungsignal kann N-oder C-terminal an das Tumorantigen angehängt sein, um dessen Detektion oder Aufreinigung zu ermöglichen. Durch die N-terminale Fusion des rekombinanten Proteins mit einer Leadersequenz wie z.B. der OmpA oder pELB-Sequenz wird der Transport in den periplasmatischen Raum ermöglicht, wo das Virus-"Assembly" stattfindet. Durch Phagemid-Rescue mit Helferphagen wie z.B. M13K07 oder VCSM13 werden Hybridphagen produziert, welche die Tumorantigene als Fusion mit einem Hüllprotein auf ihrer Oberfläche tragen. Durch die Hybridanordnung finden sich neben rekombinanten Hüllproteinen auch normale Hüllproteine in den Phagen, wodurch das Virus-Assembly und damit die Expression von größeren Proteinen auf der Phagenoberfläche erst ermöglicht wird. Die Menge der auf der Oberfläche exprimierten Tumorantigene kann durch Induktion des Promotors optimiert werden, so daß die maximale Expression eingestellt wird, bei der es gerade noch nicht zu einer Störung des Virus Assembly kommt. Beispiele eines solchen Promotors können der P_{Lac}-, der Pₜₑₜ-, P_{BAD}-Promotor sein, wobei auch andere Promotoren in Frage kommen.

In einer alternativen Ausführungsform wird ein λ-ZAP®-Bakteriophagen-System als Vektorsystem eingesetzt, wie es im US-Patent 5,128,256, (Huse, Sorge, Short) beschrieben ist. Nach einem Verpackungsprozeß mit im Handel erhältlichen Verpackungsextrakten (z.B. GigaPack®, Fa. Stratagene) und Amplifikation der Bakteriophagen erfolgt eine Transformation in filamentöse Phagen durch Koinfektion mit M13-Helferphagen. Das zugrunde liegende Prinzip ist hierbei, daß die λ-Bakteriophagen-Vektoren in sich einen Plasmid- bzw. einen Phagemidvektor tragen, der durch Koinfektion mit Helferphagen aus dem Phagenvektor herausgeschnitten werden kann. Dieser Vorgang wird in vivo-Exzision genannt und wurde erstmals von Short et al. 1988 (Nucleic Acids Res. 16: 7583) und Short und Sorge 1992,(Methods Enzymol. 216:495-508). beschrieben. Eine effiziente Exzision dieser Phagemide ist möglich bei gleichzeitiger Koinfektion einer E. coli-Zelle mit λ-ZAP® und filamentösen Helferphagen. Nach erfolgter Infektion der Bakterien wird die in die Zelle transduzierte ssDNA des filamentösen Phagen schnell in dsDNA umgeschrieben. Unmittelbar darauf beginnt die Expression von Phagenproteinen im Wirtsorganismus, wodurch die Vervielfältigung der Phagen beginnen kann. Durch dieses Verfahren ist es möglich, für individuelle Tumorzellen spezifische Tumorantigene so zu exprimieren, daß sie als lösliche Proteine oder als Fusionsproteine auf der Phagenoberfläche gebunden vorliegen.

In einer weiteren bevorzugten Ausführungsform des Verfahrens der vorliegenden Erfindung sind die Nucleinsäuren cytosolische RNAs.

In einer anderen bevorzugten Ausführungsform des Verfahrens ist das tumorspezifische Antigen mit einer Erkennungssequenz fusioniert.

Ferner betrifft die Erfindung die Verwendung eines wie oben charakterisierten Phagen zur Herstellung eines Arzneimittels zur Induktion einer spezifischen Immunantwort, vorzugsweise zur spezifischen Immuntherapie einer Neoplasie, in einem Vertebraten.

Die Erfindung betrifft auch Verfahren zur Induktion einer spezifischen Immunantwort, vorzugsweise zur spezifischen Immuntherapie einer Neoplasie, in einem Vertebraten, wobei man einem Vertebraten einen wie oben charakterisierten Phagen oder die erfindungsgemäße Zusammensetzung verabreicht.

Dabei ist die Neoplasie in einer bevorzugten Ausführungsform eine Hämoblastose, vorzugsweise ein malignes Lymphom oder eine Leukämie, ein malignes Melanom, ein urogenitales Karzinom, vorzugsweise ein Nieren-, Blasen-, Prostata- oder Hodenkarzinom, ein gynäkologisches Karzinom, vorzugsweise ein Mamma- Ovarial-, Uterus- oder Zervixkarzinom, ein gastrointestinales Karzinom, vorzugsweise ein Ösophagus-, Magen-, Kolon-, Rektum-, Pankreas- Gallenblasen-, Gallengang- oder hepatocelluläres Karzinom, ein bösartiger endokrinologischer Tumor, vorzugsweise ein Schilddrüsenkarzinom, ein maligner Lungentumor, vorzugsweise ein Bronchialkarzinom oder Mesotheliom, ein Sarkom oder ein ZNS-Tumor.

In einer weiteren bevorzugten Ausführungsform der erfindungsgemäßen Verwendung bzw. des erfindungsgemäßen Verfahrens ist der Vertebrat ein Säuger, vorzugsweise ein Mensch.

Schließlich ist in einer zusätzlichen bevorzugten Ausführungsform der erfindungsgemäßen Verwendung oder des erfindungsgemäßen Verfahrens das Arzneimittel als Injektionspräparat, vorzugsweise zur subkutanen, intradermalen, perinodalen, intravenösen, peritonealen oder intramuskulären Applikation, oder in einer per os, rektal, intrabronchial oder intranasal zu verabreichenden Form hergestellt.

Die in der vorliegenden Beschreibung zitierten Literaturstellen sind hiermit per Interpretation Teil der Beschreibung.

Die Figuren zeigen:

### Figur 1:

(a) Agarose-Gelelektrophorese der PCR-Produkte der Idiotypgene der Zellinie Daudi (z.B. erhältlich von der ATCC) mit spezifischen Primern für die schweren Ketten (V_{H}und J_{H}-Primer). (b) Entsprechende Darstellung der V_{K}- und J_{K}-Primer. Zur Größenbestimmung der Fragmente wurde der Molekulargewichtsmarker VI der Fa. Boehringer Mannheim verwendet.

### Figur 2:

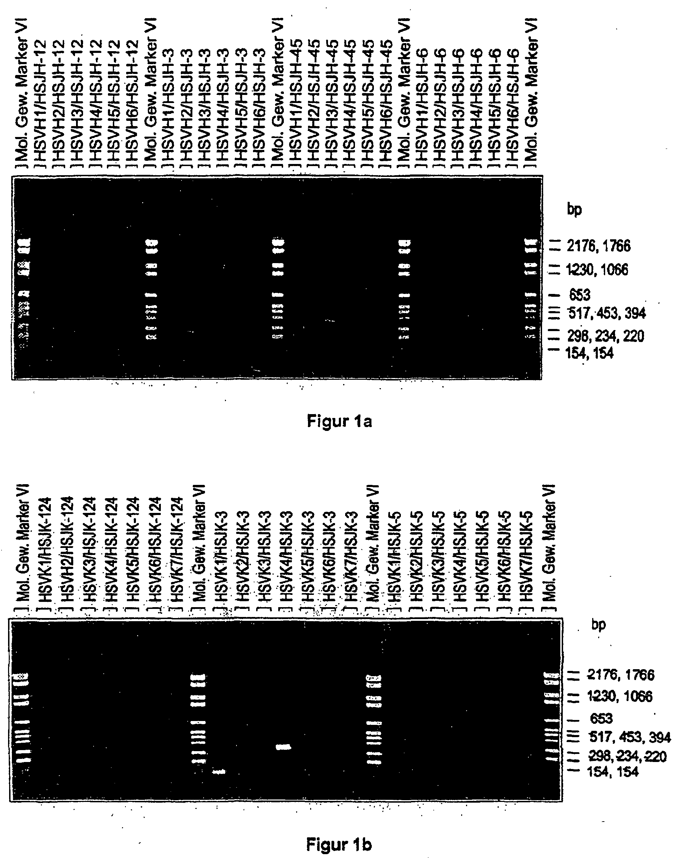

Assembly-PCR von Daudi V_{H}- und V_{K}-Fragmenten. Die aufgereinigten V_{H}- und V_{K}-Fragmente werden bei der Assembly-PCR über einen single-chain Fv (scFv) (Gly₄Ser)₃-Linker miteinander verknüpft, der komplementär zu dem 3'-Ende des J_{H}-Gens und dem 5'-Ende des V_{L}-Gens ist. Die Marker entsprechen denen der Figur 1.

### Figur 3:

Western Blot-Analyse der auf filamentösen Phagen exprimierten scFv/cpIII Fusionsproteine mit einem anti-Kappa Antikörper und SDS-PAGE-Elektrophorese von scFv/cpIII nach Aufreinigung über M1-Affinitätssäulen. Für die M1-Affinitätssäulenchromatographie wurden die ins Medium freigesetzten löslichen scFv/cpIII Fusionsproteine aus der Amplifikation von rekombinanten SurfZAP®-Phagen in calciumhaltigem Proben-Puffer aufgetragen. In der Western Blot-Analyse mit anti-Kappa Antikörpern und nach M1-Affinitätschromatographie, läßt sich das scFv/ cpIII-Fusionsprotein als ca. 48Kd großes Protein nachweisen.

### Figur 4:

Skizze der in den Beispielen verwendeten Phagemidvektoren, wobei Figur 4a den Surfscript®-Vektor (Stratagene) und Figur 4b das cpVIII-Script darstellt.

### Figur 5:

Zytotoxizitätsassay: Mit Bcl2-Phagen, Daudi-Id-Phagen und Wildtyp-Kontrollphagen gepulste dendritische Zellen (DC) wurden verwendet, um eine primäre T-Zell Antwort zu induzieren. Als Kontrolle wurden auch ungepulste DC's verwendet. Die nach 48h mit DC's stimulierten T-Zellen wurden in unterschiedlichen Verhältnissen zu den Tumorzellen gegeben und die Zytotoxizität nach 3 Tagen gemessen. Es zeigt sich eine deutliche Lyse bei BCI-2. Phagen, während Daudi Id Phagen keine Lyse induzieren.

Die Beispiele erläutern die Erfindung.

### Beispiel 1:

Bei diesem Beispiel wird das Bcl-2 Antigen kloniert und in Fusion mit Hüllprotein gIII und gVIII auf der Phagenoberfläche exprimiert. Die verwendeten Phagemidvektoren sind in Figur 4 skizziert, wobei als gIII-Protein eine verkürzte Form des gIII-Protein verwendet wird, wie sie im SurfScript-Vektor (Fa. Stratagene) beschrieben ist. Das gVIII-Protein bestand aus der vollständigen Sequenz des in filamentösen Phagen nachweisbaren Proteins. In beide Hüllproteine wurde es jeweils N-terminal fusioniert, wobei die Restriktionsstellen Notl und Spel zur Klonierung dienten. Zur Amplifizierung von Bcl-2 wurde die Plasmid-DNA des Plasmid PB4 aus E.coli 79804 (ATCC) präpariert und in eine PCR-Reaktion eingesetzt. Primer für die Amplifikation von Bcl-2 wurden aus der von Tsujimoto publizierten Bcl-2 Sequenz, Genbank M13994 abgeleitet (Tsujimoto und Croce, 1986). Die für die Amplifikation von Bcl-2 verwendeten Primer HSBCL2-FOR und HSBCL2-BACK entsprechen der Nukleinsäure 1459-1478 bzw. revers komplementären Sequenz 2159-2182 aus der vorstehend genannten publizierten Arbeit. Die Amplifikation von Bcl-2 wurde in einer 50 µl PCR-Reaktion mit 1 x PCR-Puffer (10 mM Tris-HCI, pH 8.8, 1.5 mM MgCI, 25 mM KCI) (Stratagene), 0.2 mM dNTP's, 2.5 pmol Primer und 2.5 U Taq-Polymerase (Boehringer Mannheim) in einem OmniGene Thermocycler (Fa. Hybaid) durchgeführt. Nach 3 min initialer Denaturierung wurde das Bcl-2 Gen über 30 Zyklen (1 min 94°C, 1 min 55°C, 1 min 72°C) und einem terminalen Extensionszyklus (8 min 72°C) amplifiziert. Das entstandene Bcl-2 PCR Produkt ließ sich als 0.7 Kb Fragment in einem 1.5% Agarosegel darstellen. In einer Anschluß-PCR wurden Restriktionsstellen eingebaut, so daß es im Leserahmen mit dem Hüllprotein in den gIII-bzw. gVIII Phagemidvektor kloniert werden konnte. Die BCL-2 exprimierenden Hybridphagen wurden wie im nachfolgenden Methodenteil beschrieben hergestellt. Der Nachweis der Expression des BCL-2 Proteins als Fusionsprotein mit den Hüllproteinen des filamentösen Phagen erfolgte in einer Westernblot-Analyse.

### Beispiel 2:

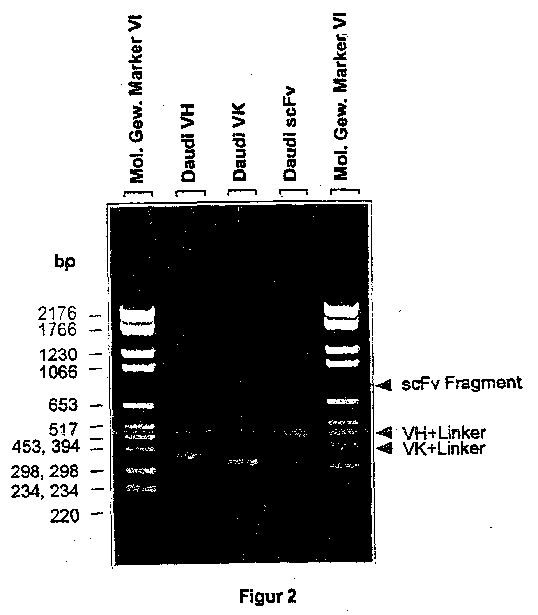

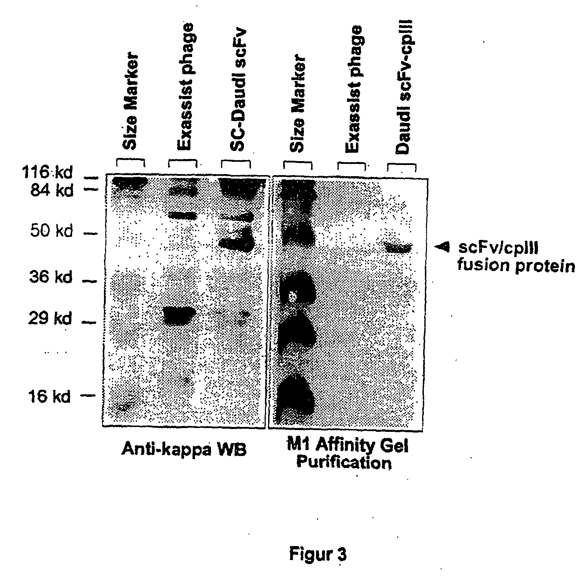

Bei diesem Beispiel werden lymphomspezifische Idiotyp-Gene kloniert und in Fusion mit cplll auf der Phagenoberfläche exprimiert. Zunächst wurde aus der Lymphomzelle Daudi nach bekannten Verfahren zytoplasmatische RNA isoliert. Die RNA wurde dann mit (dT)₁₅ Primern in cDNA umgeschrieben. Die Herstellung einer Einzelstrang cDNA reichte für eine RT-PCR aus. Die RT-PCR Reaktion wurde unter Standardbedingungen in einem OmniGene Thermocycler (Fa. Hybaid, c/o MWG-Biotech, Ebersberg) durchgeführt, wobei zur Amplifikation familienspezifische humane VH und VK-Primer (s. Tabelle 1) verwendet wurden. Im Anschluß an die RT-PCR Reaktion wurde eine Agarose-Gel-Elektrophorese zur Analyse der Amplifikationsprodukte, d.h. der Gene für die schwere Kette (V_{H}) und die leichte Kette (V_{L}) durchgeführt (s. Figur 1). Nach Extraktion der entsprechenden VH und VK-Bande aus dem Agarose-Gel wurde mit Geneclean® (Bio 101 Inc, La Jolla, CA, USA) aufgereinigt und unter den Bedingungen wie im Methodenteil beschrieben in einer Assembly-PCR zu einem Einzelstrang (single chain Fv) verknüpft. Die dabei verwendeten Primer sind aus Tabelle 2-4 zu entnehmen. Bei der Assembly-PCR werden gleichzeitig Erkennungssequenzen für Restriktionsendonukleasen in das scFv-Fragment für die anschließende Klonierung eingefügt. Im Anschluß erfolgte eine erneute Analyse des PCR-Produkts durch eine Agarose Gelelektrophorese. Anschließend wurden die scFv-Fragment mit entsprechenden Restriktionsenzymen verdaut und in den g3-Script Vektor ligiert. Vergleichsweise erfolgte auch eine Ligation in den SurfZAP-Vektor (Fa. Stratagene). Der SurfZAP-Vektor wurde in vitro nach Angaben des Herstellers verpackt (s. Methodenteil). Nach in vivo Exzision (s. Methodenteil) wurden filamentöse Phagenhybride mit dem Daudi-Idiotyp auf der Oberfläche durch Phagemid Recue hergestellt, wie im Methodenteil beschrieben. Zum immunchemischen Nachweis der exprimierten Idiotyp-Proteine wurden die Phagenproteine auf SDS-Polyacrylamidgelen aufgetrennt. Der Western Blot bewies das Vorhandensein der exprimierten Idiotyp-Proteine (siehe Figur 2) Es konnten auch überexprimierte lösliche TA-Fusionsproteine über M1-Affinitätssäulen aufgereinigt werden, wobei die Phagen zunächst 1:1 in einer Pufferlösung (50mM Tris-HCI, 0,15 M NaCl, 1 mM CaCl₂, 5% Triton X-100, pH 7,0) verdünnt wurden. Anschließend wurde die voräquilibrierte M1-Säule (Fa. Kodak) mehrmals sequentiell mit 5 ml Glycin-HCl-Lösung (0,1 m Glycin-HCI, pH3,0) gespült und zuletzt mit 5 ml TBS äquilibriert. Die Affinitätssäule wurde in Anwesenheit von 1 mM CaCl₂ mit den löslichen TA geladen. Die Säule wurde dreimal mit 12 ml TBS/Ca (TBS-Puffer + 1 mM CaCl₂) gewaschen und die an die Säule gebundenen Proteine sechsmal mit 1 ml Glycin-HCl-Puffer eluiert. Zur Neutralisation wurde Tris-HCI, pH 8,0 zugesetzt. Nach Waschen mit TBS-Puffer wurden die eluierten Proteine im Polyacrylamidgel analysiert.

### Beispiel 3:

Dieses Beispiel zeigt die lymphomspezifischen Idiotypgene der Zellinie Daudi in Fusion mit cpVIII auf der Phagenoberfläche exprimiert. Die Isolierung, Amplifikation und Assembly-PCR der Idiotypgene erfolgte wie in Beispiel 2. Die so gewonnenen, patientenspezifischen scFv wurden nach Restriktionsverdau in den cpVIII-Script-Phagemidvektor kloniert und Phagen wie im Methodenteil beschrieben über Phagemid Rescue produziert. Die auf der Oberfläche von Phagen exprimierten Daudi-Idiotypproteine wurden in einer Westernblotanalyse nachgewiesen.

### Beispiel 4:

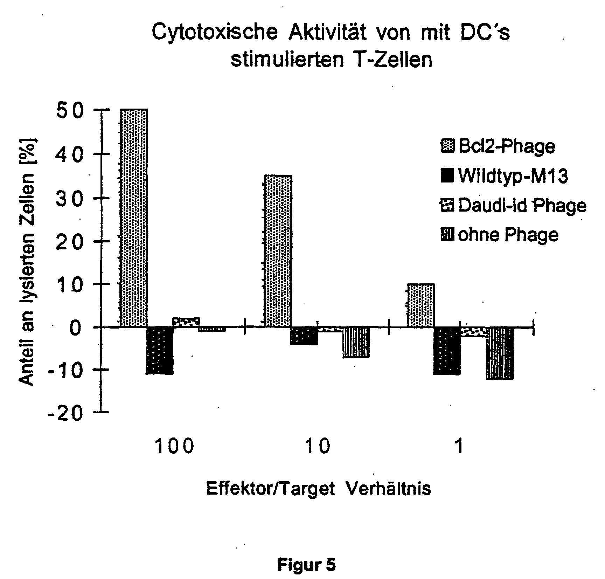

Dieses Beispiel zeigt in vitro Daten, durch die eine zelluläre Immunantwort durch in Beispiel 1-3 produzierte Phagenvakzinen nachgewiesen werden kann. Es wurden dendritische Zellen wie im Methodenteil beschrieben präpariert und am Tag 5 der Umdifferenzierung mit den Phagen-Idiotypen gepulst. Dazu wurden zunächst DC's gesplittet und mit Medium gewaschen. Anschließend erfolgte die Resuspension in DMEM/10% FCS zu einer Konzentration von 2 x 10⁶ Zellen pro ml Medium. In einer 24-well Platte wurden dann 500 µl (5 x 10⁵) DC's per well vorgelegt. Diese wurden zur Antigenbeladung mit 50-500 µl Phagensuspension (1 x 10¹⁰ - 1 x 10¹¹ pfu/ml) über mehrere Stunden (mind. 2h) gepulst. Die so stimulierten DC's wurden in Proliferationsassays und Zytotoxizitätsexperimenten eingesetzt. T-Zell Proliferationsassays wurden in 96-weil Platten durchgeführt. Dabei wurden 5 x 10⁴ T-Zellen und 5 x 10³ bestrahlte dendritische Zellen pro well eingesetzt, was einem Effektor/ Stimulationsverhältnis von 10:1 entspricht. Von jeder Versuchsanordnung wurden Triplikate angelegt. Die Antigen-beladenen DC's wurden auf eine Konzentration von 1 x 10⁵ Zellen/ml in Iscove's Medium/10% FKS eingestellt und mit 4000 rad bestrahlt, um zu vermeiden, daß der ³H-Thymidineinbau der proliferierenden T-Zellen durch evtl. proliferierende DC's überlagert wird. Nach der Bestrahlung wurden die DC's 1:1 verdünnt. Autologe T-Zellen wurden auf eine Konzentration von 1 x 10⁶ Zellen/ml eingestellt. Davon wurden nach 1:1 Verdünnung in Iscove's Medium 100 µl in die entsprechenden wells vorgelegt. Wells, die nur DC's oder T-Zellen enthielten, wurden mit 100 µl Iscove's Medium auf 200 µl aufgefüllt. Zur submaximalen Stimulation der Zellen wurden in alle Löcher 10 µl ConA-Lösung (10 µg/ml in Iscove's Medium) pipettiert, mit Ausnahme der Löcher, bei denen eine maximale Stimulation der T-Zellen mit PHA (50-100 µg/ml) vorgesehen war. Nach 48 h, 72h und 96h wurde 25 µl [³H]-Thymidin (25 µCi/ml) pro well zugegeben und der Thymidineinbau für 18 h bestimmt. Im Anschluß an die 18 h wurde die 96-well Platte bei -80°C eingefroren. Die 96-well Platten wurden im Harvester geerntet und die Glasfibermembranen anschließend im Wallac 1460 Scintilation Counter gemessen. Eine Proliferation der T-Zellen wurde sowohl bei BCI-2-Phagen als auch bei Daudi-Idiotyp-Phagen nachgewiesen. Zur Messung der Zytotoxizität wurden 1 x 10⁶ T-Zellen mit 5 x 10⁴ antigenbeladenen DC's in einem Verhältnis von 1:20 (Stimulator:Target) für 48 h aktiviert. In Bezug auf T- und Stimulatorzellen wurden autologe Zellen vom selben Spender verwendet. Als Medium wurde Iscove's Medium mit 20 U/ml IL-2 und 5% FCS verwendet. Die IL-2 aktivierten T-Zellen wurden anschließend für zytotoxische Experimente eingesetzt. Hierzu wurden T-Zellen und allogene Lymphomzellen (Daudi bzw. die Lymphozelle HOL welche eine typische Lymphomzelle eines CBCC Non-Hodgkin-Lymphoms darstellt.) in unterschiedlichen Konzentrationen (10:1, 1:1, 0.1:1) zusammen inkubiert. Anschließend wurde der Anteil der lysierten Lymphomzellen in Standardverfahren gemessen. Das Ergebnis ist in Fig. 5 sichtbar und zeigt, daß sich eine spezifische Lyse durch BCL-2-Phagen induzieren läßt, während im Kontrollexperiment mit Daudi-ldiotyp-Phagen infolge der fehlenden Expression der Histokompatibilitätsantigene auf Daudi-Zellen keine Lyse zeigt.

### Methoden:

Die folgenden von den Anmeldern verwendeten Methoden sind in Sambrook, J., E. F. Fritsch, and T. Maniatis (ed.). 1989. Molecular Cloning, A Laboratory Manual, 2nd Edition ed., Cold Spring Harbor Laboratories, Cold Spring Harbor, NY. beschrieben: DNA-Schnellaufschluß, Restriktionsverdau, Ligation, Agarose Gel Elektrophorese, Immunblotting, Bakterienkultur mit unterschiedlichen Medien und Polyacrylamidgelelektrophorese und deswegen nicht näher beschrieben.
Restriktionsenzyme und Ligasen wurden von Boehringer Mannhein und New England Biolabs verwendet und entsprechend den Herstellerangaben eingesetzt.
Den Stamm E. coli 79804, welches das Plasmid PB4 mit dem BCL-2 Gen trägt, erhielten wir von der American Type Culture Collection (ATCC, Rockville, MD, USA). Die universelle verfügbare Zellinie Daudi wurde aus unserer Stammsammlung entnommen, ebenso die Lymphomzelle HOL. Die Experimente können ebenso mit anderen Krebszellen/Krebszellinien, die entsprechende Eigenschaften aufweisen und dem Fachmann zur Verfügung stehen bzw. ohne weiteres isoliert werden können, durchgeführt bzw. wiederholt werden.

### RNA-Präparation modifiziert nach Favaloro:

Es wurden Lymphomzellen der Zellinie Daudi verwendet. Dazu wurden 1-2 Kryoröhrchen mit ca. 2 x 10⁷ Zellen aufgetaut und mit 10 ml McCoy-Medium verdünnt. Die Zellen wurden bei 300 x g abzentrifugiert und dreimal mit 5 ml kalter PBS-Lösung (120 mM NaCI, 2,7 mM KCI, 10 mM K-Phosphat Puffer, pH 7,4, 4°C) gewaschen. Das Zellpellet wurde dann in 375 µl RNA-Extraktionspuffer (50 mM Tris-HCI, pH 8,0, 100 mM NaCI, 5 mM MgCl₂, 0,5 % (v/v) Nonidet P-40, 1 mM DTT, 1000 U/ml RNAsin, 4°C) aufgenommen und solange auf- und abpipettiert, bis eine komplette Lyse auftrat. Anschließend wurde im Eisbad inkubiert, abzentrifugiert und der Überstand mit 4 µl 20 % SDS versetzt. Schließlich wurde die RNA zweimal mit Phenol-Chloroform-Isoamylalkohol (25:24:1) extrahiert. Durch Zugabe von 0,1 Vol. 3 M Na-Acetat, pH 5,2 und 2,5 Vol. eiskaltem Ethanol wurde die RNA über Nacht bei - 20°C oder 30 min bei -80°C präzipitiert. Nach Zentrifugation und einem Waschschritt wurde das RNA-Pellet in 50 µl DEPC-H₂0 aufgenommen.

### cDNA-Synthese modifiziert nach Gubler und Hoffmann:

Für eine cDNA-Synthese Reaktion wurde 1-2 µg zytoplasmatische RNA verwendet, welche mit ddH₂0 auf ein Volumen von 8 µl gebracht und zur Beseitigung von störenden Sekundärstrukturen 10 min bei 65°C inkubiert und anschließend auf Eis gestellt wurde. Währenddessen wurde der cDNA Reaktionsmix hergestellt, welcher pro Reaktionsansatz 2 µl Reaktionspuffer (100 mM Tris-HCI, 500 mM KCI, pH 8.3), 4 µl 25 mM MgCl₂, 2 µl 10mM dNTP-Mix, 2 µl Oligo-(dT)₁₅ Primer (0.8 µg/µl), 50 U RNase Inhibitor (Boehringer Mannheim) und 20 U AMV-Reverse-Transcriptase (AMV-RT, Boehringer Mannheim) enthielt. Die denaturierte RNA wurde mit dem cDNA Reaktionsmix 10 min bei 25°C inkubiert, damit die Oligo-dT Primer an die RNA hybridisieren. Anschließend wurde der 1. Strang cDNA während einer Inkubation von 1 h bei 37°C geschrieben. Nach der cDNA Synthese wurde die AMV-RT 5 min bei 94°C deaktiviert. Die einzelsträngige cDNA wurde entweder sofort in die PCR eingesetzt oder bis zur weiteren Verwendung bei -20° gelagert.

### Standardbedingungen der PCR:

Für eine Standard-PCR wurden 100 ng Matrix-DNA eingesetzt. Pro 100 µl Reaktionsansatz waren 1 x Taq-Puffer (10 mM Tris-HCI, 50 mM KCI, pH 8.3), 0.2-0.02 mM dNTP-Mix, 1.5 mM (1-2.5 mM) MgCl₂, je 20 pmol Primer und 2.5 U Taq-Polymerase enthalten. In der Standardreaktion wurden 25-30 Zyklen mit 1 min bei 94°C (Denaturierung), 1 min bei Annealingtemperatur (Primer-Schmelztemperatur minus 5°C) und 1 min bei 72°C (Polymerisation) durchgeführt. Zur Amplifikation von DNA-Fragmenten kann anstelle von Taq-Polymerase auch eine "proof-reading" Polymerase wie z.B. Pwo-Polymerase verwendet, welche deutlich weniger Fehler macht als die Taq-Polymerase.

### RT-PCR Reaktion:

In die RT-PCR Reaktion wurde 5-20 µl cDNA eingesetzt. Der PCR-Reaktionsmix enthielt bei einem Gesamtvolumen von 100 µl pro Reaktion 8 µl PCR-Puffer (identisch mit cDNA Reaktionspuffer), 2 µl 25 mM MgCl₂, 1 µl Gelatine 0.05%, 0.5 µl Taq DNA-Polymerase (5 U/µl) und je 1,5 µl Upstream- und Downstream-Primer (20 pmol/µl). (Die Sequenzen der verwendeten Primer befinden sich im Anhang). Zur Vermeidung der Kondensation wurde der RT-PCR Ansatz mit 2 Tropfen Mineralöl (Fa. Sigma Chemical, St. Louis. MO, USA) überschichtet. Die PCR-Reaktion wurde im OmniGene Thermocycler (Fa. Hybaid, c/o MWG-Biotech, Ebersberg) bei folgenden Amplifikationsbedingungen durchgeführt:

| *Initialer Zyklus* | | |
|---|---|---|
| Denaturierung | 94°C | 4 min |

| *Amplifikationszyklen, 30 Zyklen* | | |
|---|---|---|
| Denaturierung | 94°C | 1 min |
| Annealing | 50-60°C | 1 min |
| Extension | 72°C | 1 min |

| *Extensionszyklus* | | |
|---|---|---|
| Extension | 72°C | 8 min |

Im Anschluß an die RT-PCR Reaktion wurde eine Agarosegelektrophorese zur Analyse der Amplifikationsprodukte durchgeführt. Schwache Banden konnten durch Anfärbung mit SYBR™-Green (FMC Bioproducts) besser sichtbar gemacht werden. Anstelle von Taq-Polymerase wurde teilweise auch eine proof-reading Polymerase wie die Pwo-Polymerase verwendet, in diesem Fall mußte anstelle von MgCl₂ dann MgSO₄ der PCR-Reaktion zugesetzt werden.

### Standardbedingungen für die Assembly ―PCR von Idiotypgenen:

Für die Assembly-PCR wurden mindestens 100 ng der Idiotyp-PCR Fragmente bzw. der schweren und leichten Kette eingesetzt, da die Ausgangsmenge der primären DNA-Fragmente für den erfolgreichen Ausgang der Assembly-PCR entscheidend ist. Zur Entfernung der noch vorhandenen Primer wurden Idiotyp-PCR-Produkte mit Geneclean (Bio 101 Inc., La Jolla, Ca, USA) aufgereinigt. Je 100-500 ng der beiden DNA-Fragmente wurden in die Reaktion eingesetzt. Eine etwa äquimolare Menge Linker-Primer wurde eingesetzt. Der Assembly-PCR-Reaktionsmix enthielt bei einem Gesamtvolumen von 50 µl pro Reaktion 5µl 10 x PCR-Puffer (100 mM Tris-HCI, 500 mM KCl, pH 8.3), 3 µl 25 mM MgCl₂ (MgSO₄), je 100-500 ng amplifizierte V_{H} und V_{K/λ}, 2.5 U Taq (Pwo-) DNA-Polymerase und je 2 µl Linker-Primer (25 pmol/ µl). Die Sequenzen der verwendeten Linker-Primer befinden sich in Tabelle II. Die Assembly-PCR-Reaktion wurde mit 2 Tropfen Mineralöl überschichtet. Die Amplifikationsbedingungen für die Assembly-PCR waren: ein initialer Zyklus mit 94°C ― 4 min, zehn sich wiederholende Zyklen mit 94°C ― 1 min, 60°C ―2 min, 72°C ―1 min. Direkt danach wurde zur Amplifikation der variablen Einzelstrangfragmente (scFv) eine Anschluß-PCR ("pull through" -PCR) mit Megaprimern durchgeführt. Dabei wurden degenerierte Primer verwendet, welche die scFv jeweils außen flankierten und zusätzlich Sequenzen für Restriktionsendonukleasen, pelB-Leader Sequenz und ein Flag-Protein enthielten. Für die PCR mit diesen Megaprimern wurde ein 50 µl Mastermix hergestellt. Darin waren enthalten: 5µl 10 x PCR-Reaktions-Puffer #5 (Fa. Stratagene, La Jolla, Ca, USA), 5µl Formamid 50%, 1µl 10mM dNTP's, je 20pmol Außenprimer, 2.5U Taq (Pwo) DNA-Polymerase. Zu diesem Ansatz wurde der Assembly-PCR-Ansatz pipettiert und alles mit Mineralöl überschichtet. Diese Pull-through-PCR wurde unter folgenden Amplifikationsbedingungen durchgeführt:

| *Initialer Zyklus* | | |
|---|---|---|
| Denaturierung | 94°C | 1 min |

| *Amplificationszyklen, 5 Zyklen* | | |
|---|---|---|
| Denaturierung | 94°C | 1 min |
| Annealing | 50°C | 2 min |
| Extension | 72°C | 1 min |

| *Amplificationszyklen, 30 Zyklen* | | |
|---|---|---|
| Denaturierung | 94°C | 1 min |
| Annealing | 55°C | 2 min |
| Extension | 72°C | 1 min |

| *Extensionszyklus* | | |
|---|---|---|
| Extension | 72°C | 8 min |

Im Anschluß an die Anschluß-PCR-Reaktion wurde eine Agarosegelelektrophorese zur Analyse der entstandenen PCR-Produkte durchgeführt.

### Verpackung von λ-Bakteriophagen

Zur Verpackung der λ-Bakteriophagen-DNA wurden Verpackungsextrakte der Fa. Stratagene, Heidelberg verwendet. 1 µg ligierte Phagen-DNA wurden in Freeze-Thaw Extrakt kurz auf Eis inkubiert. Anschließend wurden 15 µl des aufgetauten Sonic Extrakts dazugegeben. Die eigentliche Verpackungsreaktion erfolgte dann während einer zweistündigen Inkubation bei 22°C. Die Reaktion wurde durch Zugabe von 500 µl SM-Puffer (100 mM NaCI, 0,2 % MgS0₄, 50 mM Tris-HCI, pH 7,5, 2 % g/v Gelatine) abgestoppt und die Proteine durch Zugabe von Chloroform präzipitiert. Nach kurzer Zentrifugation wurde der Überstand in ein sauberes Eppendorfgefäß überführt und bei 4°C bis zur Amplifikation gelagert. Nach der Verpackung wurde der Titer der hergestellten infektiösen Phagenpartikel bestimmt.

### Amplifikation der SurfZAP®-λ-Phagen

20 ml TB-Medium (LB + 0,2 % g/v Maltose + 10 mM MgSO₄) wurde mit einer Bakterienkolonie von einer frischen Über-Nacht-Kultur überimpft und 4-6 Stunden im Schüttelinkubator bis zur späten midlog-Phase inkubiert. Nach Zentrifugation wurden die Bakterien in 10 mM MgS0₄ resuspendiert und eine OD₆₀₀ von 0,5 eingestellt. 600 µl dieser Bakteriensuspension wurden mit 5 x 10⁴ verpackten Phagen infiziert. Zur Absorption der Phagen erfolgte eine Inkubation von 10 min im Schüttelinkubator bei 37°C. Anschließend wurden die Bakterien mit warmen Top-Agar vermischt und auf eine 150 mm Agarplatte ausplattiert. Die ausplattierten Agarplatten wurden 6-8 Stunden bei 37°C kultiviert und anschließend mit einem SM-Puffer beschichtet. Die Agarplatten wurden dann über Nacht bei 4°C unter Schwenken inkubiert. In dieser Zeit diffundierten die amplifizierten Phagen in den Puffer und konnten durch Absaugen des Puffers aufgefangen werden. Die amplifizierten Phagen wurden kurz zentrifugiert und unter Zugabe von 0,3 % Chloroform bei 4°C gelagert.

### In vivo-Exzision und Amplifikation von SurfScript®-Phagen in SOLR®-Zellen

Für die in vivo-Exzision wurden XL-1 Blue und SOLR-Zellen in TB-Medium kultiviert. Nach Erreichen der midlog-Phase wurden die Bakterien zentrifugiert und in 10 mM MgS0₄ resuspendiert. Bei XL-1 Blue Zellen wurde eine OD₆₀₀ von 5,0 bei SOLR-Zellen von 1,0 eingestellt und die Zellen bis zur Verwendung bei 4°C gelagert. Die XL-1 Blue Zellen wurden mit SurfZAP®-Phagen infiziert. Anschließend wurden die Bakterien 1:1 mit ExAssist®-Helferphagen superinfiziert. Zur Absorption der Phagen wurden die Bakterien bei 37°C im Schüttelinkubator unter leichtem Schwenken inkubiert. Anschließend wurde 20 ml LB-Medium zu den Bakterien gegeben und die Kultur bei 37°C im Schüttelinkubator bei 150 UpM 2-3 Stunden inkubiert. Im Anschluß an die Kultur wurden die Bakterien 20 min bei 70°C inkubiert. Dadurch wurden Bakterien und λ-Phagen abgetötet. Nach Zentrifugation wurde der Überstand mit den enthaltenen SurfScript®-Phagen in ein frisches Röhrchen überführt und bei 4°C 1-2 Monate gelagert. Die Amplifikation wird mit SOLR®-Stämmen durchgeführt. Diese wurden in einem Verhältnis von 1:1 infiziert und in 100 ml LB-Medium mit Zusatz von 100 µg/ml Carbenicillin und 50 µg/ml Kanamycin bis zur midlog-Phase inkubiert. Die Bakterien wurden durch Zentrifugation pelletiert und in 10 mM MgS0₄ resuspendiert.

### Herstellung von kompetenten Bakterien (nach einer Methode von Hannahan).

Der E. coli Stamm XL1-Blue wurde auf einer LB-Agarplatte über Nacht vorkultiviert, um Einzelkolonien zu erhalten. Eine Kolonie wurde in TYM-Medium (2 % Bacto Trypton 0,5 % YEAST-Extrakt, 0,1 M NaCI und 10 mM-MgSO₄) bis zu einer OD₆₀₀ = 0,5 vermehrt. Diese Suspension wurde in 100 ml TYM gegeben und erneut bis zu einer OD₆₀₀ von 0,5 vermehrt. Anschließend wurden die Bakterien auf 0°C abgekühlt und abzentrifugiert (15 Minuten bei 4000 UpM, 4°C). Das Pellet wurde vorsichtig in 20 ml TFB-I-Lösung (30 mM KOH, 50 mM MnCl₂, 100 mM CaCl₂, 15 % Glyzerin) resuspendiert und 15 Minuten bei 0°C inkubiert. Nach erneuter Zentrifugation (3800 UpM/5 Minuten/5°C) wurde das Pellet in 4 ml TFB-II Lösung (10 mM MOPS, pH 7, 0,75 mM CaCl₂, 10 mM KCI, 15 % Glyzerin) resuspendiert und in vorgekühlte Eppendorf-Gefäße zu 100-200 µl aliquotiert und in flüssigem Stickstoff eingefroren und bei -80°C gelagert.

### Transformation von Bakterien

100 µl kompetente Zellen wurden frisch aufgetaut und 2 µl 0.5M ß-ME hinzugegeben. Ein Teil (1-5µl) eines 20 µl Ligationsansatzes wurde vorsichtig hinzugegeben und vorsichtig mit den Zellen vermischt. Es erfolgte eine 30 minütige Inkubation im Eisbad. Anschließend wurde die Suspension 45 sec. bei 42°C im Wasserbad inkubiert (Hitzeschrank) und dann erneut für 5 Minuten ins Eisbad gestellt. Die Suspension wurde dann mit 0,9 ml SOB-Medium versetzt und 60 Minuten bei 37°C geschüttelt. Ein Aliquot des Ansatzes (200 µl) wurde auf vorgewärmte LB-Agarplatten plus Ampicillin ausgestrichen und über Nacht bei 37°C inkubiert. Dabei gewachsene Klone wurden vermehrt und durch ein konventionelles Schnellaufschlußverfahren und anschließenden Restriktionsverdau analysiert.

### Phagemid-Rescue:

Es wurden filamentöse Hybrid-Phagen hergestellt, die die Tumorantigene in Fusion mit einem Hüllprotein auf der Oberfläche tragen. TG1-Klone wurden über Nacht bei 37°C auf Minimal Agarplatten (+ 100 µg/ml Ampicillin) inkubiert. Am Morgen wurde eine Impföse mit Bakterien von der Platte in 50 ml 2 x YT-AG-Medium ( 2 x YT Medium + 100µg/ml Amp und 2% Glucose) verdünnt und bis zur midlog Phase bzw. einer OD₅₅₀ₙₘ = 0.5 kultiviert. Wenn sich die Zellen in der midlog Phase befanden (OD₅₅₀ₙₘ: 0.5) wurden M13K07- oder VCSM13 Helferphagen mit einer MOI von 1:10 zu den Bakterien gegeben. Die Bakterien wurden 30 min bei 37°C unter leichtem Schütteln inkubiert. Die Bakterien wurden abzentrifugiert und in 500 ml 2 x YT-AK ( 2 x YT Medium + 100 µg/ml Amp und 50 µg/ml Kanamycin) resuspendiert. IPTG wurde in der gewünschten Konzentration (in der Regel 0.5-2 mM/ml) zugesetzt und Bakterien wurden 18h bei 30°C im Schüttler bei 180 UpM kultiviert. Die Bakterienkultur wurde dann bei 4500 x UpM 15 min bei 4°C abzentrifugiert. Der Überstand wurde in ein 1000 ml Gefäß überführt und Phagen durch Zugabe von 1/5 Vol. (125 ml) PEG/NaCl ÜN im Eisbad präzipitiert. Die Phagen wurden dann in 250 ml Corning Zentrifugenbecher überführt und bei 4000 UpM, 15 min, 4°C zentrifugiert. Das Phagenpellet wurde in 30 ml 1 x PBS resuspendiert und in Oak-Ridge Zentrifugenröhrchen (Nalgene 3115-0050) überführt und 10 min bei 15.000 UpM, 4°C in seinerm SS34 Rotor zentrifugiert. Hierdurch werden die restlichen Zell-Bestandteile der Bakterien abzentrifugiert. Die Phagen wurden anschließend durch Zugabe von 1/5 Vol. PEG/NaCl ein weiteres Mal präzipitiert und in 5ml PBS resuspendiert. Zur Pasteurisierung wurden die Phagen durch ein 0.45 µm Sterilfilter filtriert und bis zur Verwendung bei 4°C gelagert oder aliquotiert bei -20°C eingefroren.

### Endotoxinaufreinigung von Phagen mittels Triton X-114:

Die Phagensuspension wurde sterilfiltriert und mit 1/5 Vol. PEG8000/1.5M NaCI gefällt. Die Phagen wurden in 1 ml PBS resuspendiert. 1% Triton X-114 wurde zugegeben. 5 min Inkubation auf Eis bis Suspension homogen erscheint. Die Probe wurde 5 min bei 37°C inkubiert und kurz bei max. Geschwindigkeit in einer konventionellen Eppendorf Zentrifuge zentrifugiert. Dabei bilden sich 2 Phasen, wobei die obere wässrige Phase die Phagen enthält. Diese wird vorsichtig abpippettiert, ohne diese mit der unteren Phase zu kontaminieren. Diese Triton X-114 Zweiphasenseparation wird mit der wässrigen Phase noch 3 x wiederholt. Zum Schluß werden die Phagen erneut mit PEG/NaCl präzipitiert und die Phagen in PBS resuspendiert. Die Bestimmung des Endotoxingehalt erfolgte mit der LAL-Methode, wobei der Endotxingehalt üblicherweise zwischen 0.1 und 10 EU/ml liegen sollte. Die Triton X-114 Zweiphasenseparation sollte solange wiederholt werden, bis der gewünschte Aufreinigungsgrad erreicht ist.

### Isolierung von Leukozyten aus dem peripheren Blut:

Nach der Venenpunktion wurden vom Heparinblut jeweils 5 ml in ein 50 ml Falcon®-Röhrchen überführt und mit 40 ml kaltem Ery-Lysis Puffer (155 mM Ammoniumchlorid, 10 mM Kaliumhydrogencarbonat, 0.2 mM EDTA) überschichtet. Während der Lyse wurde die Lösung 10 min bei 4°C inkubiert. Anschließend wurde die Suspension zentrifugiert (1280 UpM, 10 min, 4°C) und 3 mal mit PBS Dulbecco's (Gibco/BRL, Grand Island, NY, USA) gewaschen. Das Pellet wurde in Medium aufgenommen und eine Konzentration von 1 x 10⁷ Zellen/ml eingestellt. Die Zellen wurden bis zur weiteren Verwendung unter Zusatz von 20% FCS und 10% DMSO in flüssigem Stickstoff gelagert, oder sofort aufgearbeitet.

### Isolation von mononukleären Zellen durch Dichtegradientenzentrifugation:

Heparinblut wurde mit PBS 1:1 verdünnt. In einem 50 ml Falcon®-Röhrchen wurde 20 ml 20°C warme Ficoll Separating Solution (Seromed, Biochrom AG, Berlin) vorgelegt und vorsichtig mit 20 ml PBS-Blut überschichtet. Nach Zentrifugation (2000 UpM, 20 min, RT, ohne Bremse) wurde die Interphase (enthält Monozyten, Lymphozyten und Blasten) vorsichtig mit einer Pasteurpipette abpipettiert. Die mononukleären Zellen wurden 2 x mit eiskaltem PBS gewaschen und entweder sofort weiterverarbeitet oder in Einfriermedium (450 µl RPMI, 450 µl McCOY, 10% DMSO) eingefroren.

### Magnetische Zellseparation:

Für die magnetische Zellseparation wurden die Zellen mit für die Separation ausreichend spezifischen Antikörper (10 - 20 µl / 1 x 10⁷ Zellen) in 200 µl PBS/BSA bei 4-8°C 30 min inkubiert. Zur Entfernung der überschüssigen AK wurden die Zellen 3 mal mit PBS/BSA gewaschen (2000 UpM, 4 min, RT). Anschließend wurden die mit dem primären AK gefärbten Zellen in 100 µl PBS/BSA aufgenommen und mit einem zweiten ferrochromhaltigen, sekundären AK (25 µl/ 1 x 10⁷ Zellen) für 30 min bei 4°C inkubiert. Nach erneutem Waschen wurden die so behandelten Zellen auf eine voräquilibrierte, im magnetischen Feld hängende RS⁺-Säule (Milteyni-Biotech) gegeben, so daß die markierten Zellen in der Säule magnetfixiert wurden. Die so fixierten Zellen wurden in ausreichend PBS/BSA gewaschen, während die nicht fixierten Zellen zur weiteren Verwendung in einem separaten Röhrchen aufgefangen wurden. Nach Entfernung des Magneten konnten die magnetfixierten Zellen aus der Säule durch Waschen mit PBS/BSA eluiert werden.

### Herstellung von dendritischen Zellen:

Zur Herstellung von dendritischen Zellen wurden Monozyten aus einem Buffy Coat oder peripherem Blut verwendet. Nach Dichtegradientenzentrifugation mit Ficoll-Lösung (s.o.) wurden die mononukleären Zellen in Iscove's Medium (ohne Serum) auf eine Konzentration von 5 - 10 x 10⁶ Zellen/ml eingestellt. 15 ml Zellen wurden dann in Zellkulturflaschen pipettiert und eine Stunde im Brutschrank kultiviert, um Monozyten und Lymphozyten zu trennen. In dieser Zeit kommt es zur Adhärenz der Monozyten an den Boden der Kulturflaschen. Die nichtadhärenten Lymphozyten wurden anschließend abgesaugt und zur T-Zell Präparation verwendet. Die adhärenten Monozyten wurden mehrere Male mit warmen Medium gewaschen und eine weitere Stunde im Brutschrank inkubiert. Nach erneutem Waschen wurden die adhärenten Zellen in RPMI 1640-Medium aufgenommen, das 5% FKS oder 1% autologes Serum enthielt. Im Normalfall wurden die Monozyten so auf etwa 60% angereichert. Um eine reinere CD14⁺-Population zu bekommen, wurden die adhärenten Monozyten über magnetgekoppelte AK mittels MACS (Fa. Miltenyi-Biotech, Bergisch-Gladbach) auf Werte von über 95 % aufgereinigt.
Zur Umdifferenzierung in dendritische Zellen wurden die Monozyten in RPMI 1640-Medium unter Zusatz von Pen/Strep und L-Glutamin bei 37°C, 5% CO₂ kultviert. Dem Medium wurde 800 U/ml GM-CSF und 500 U/ml IL-4 (beide Zytokine von Genzyme) zugesetzt. Alle 2-4 Tage wurde das Medium und die Zytokine erneuert. Die Reinheit der Präparation und die Schritte der Umdifferenzierung zu dendritischen Zellen wurde durch FACS-Analysen kontrolliert.

Weitere hier nicht näher spezifizierte Verfahren, Bakterienstämme, Phagenstämme und weitere Materialien sind konventioneller Art bzw. käuflich erwerbbar.

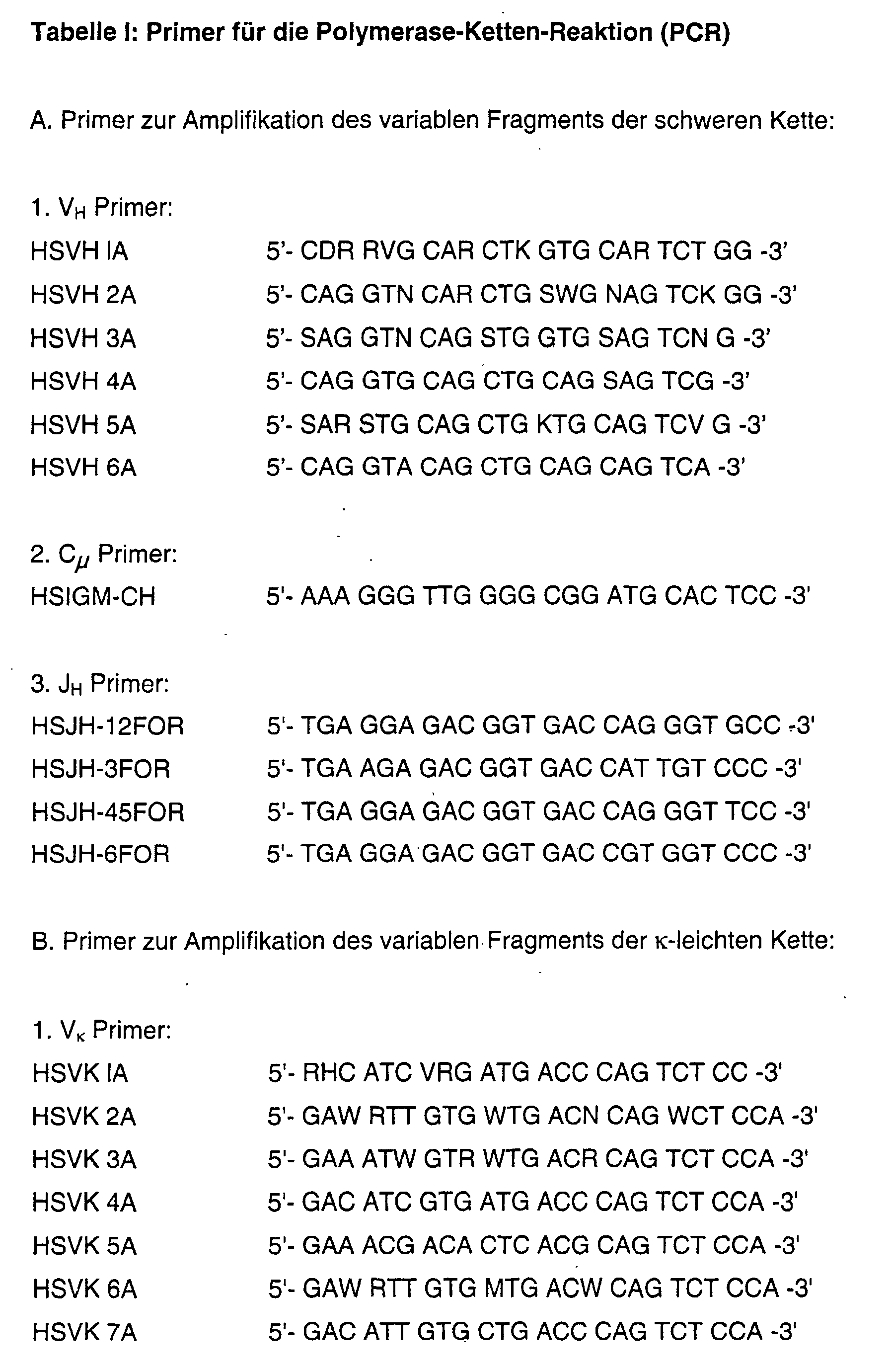

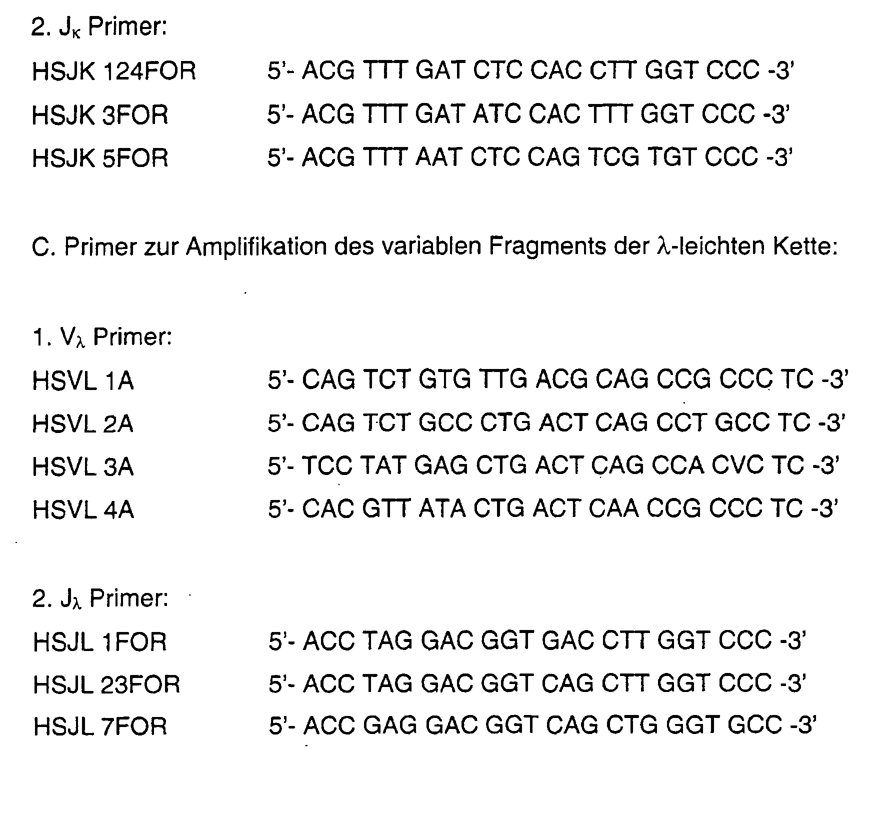

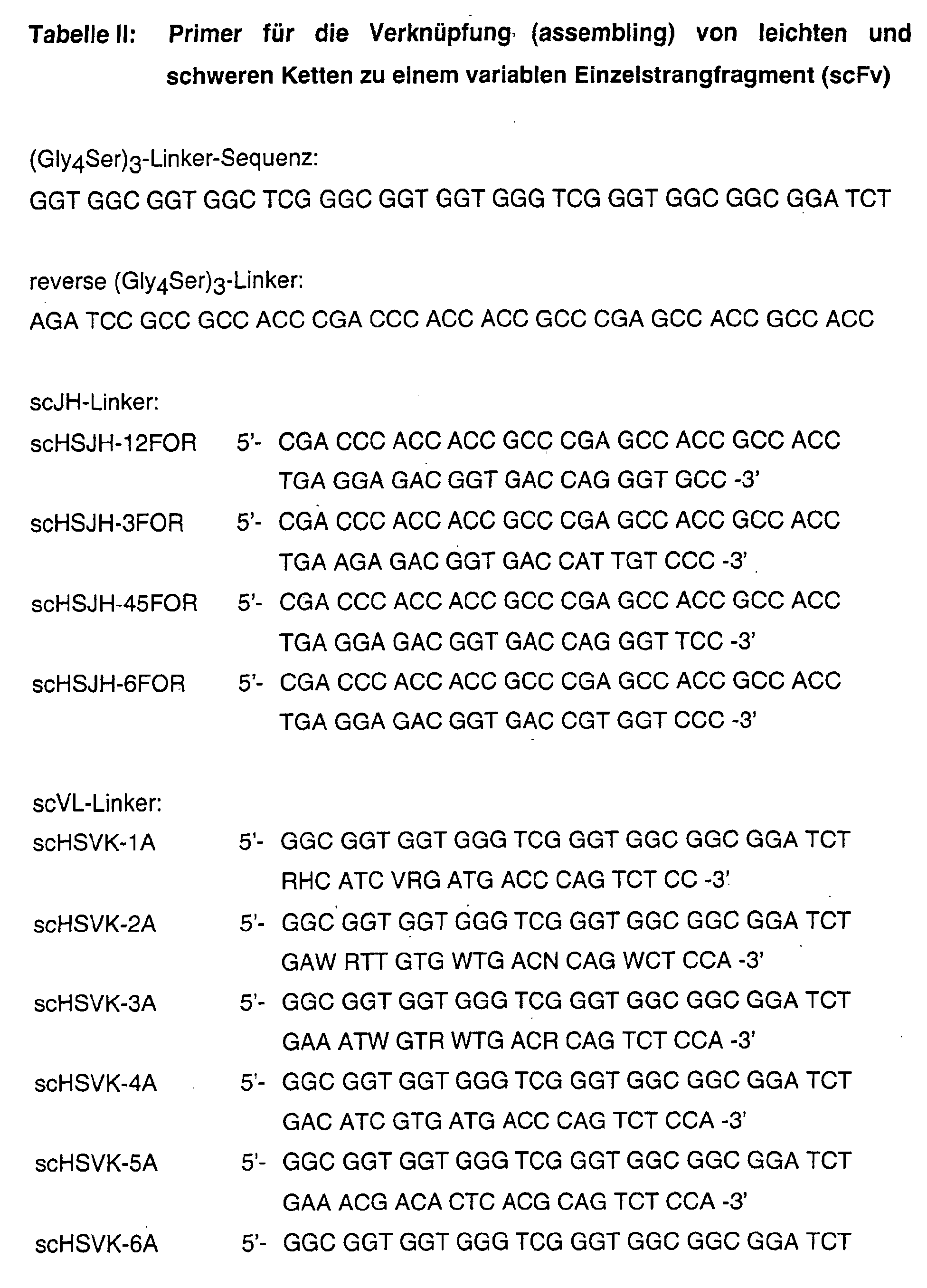

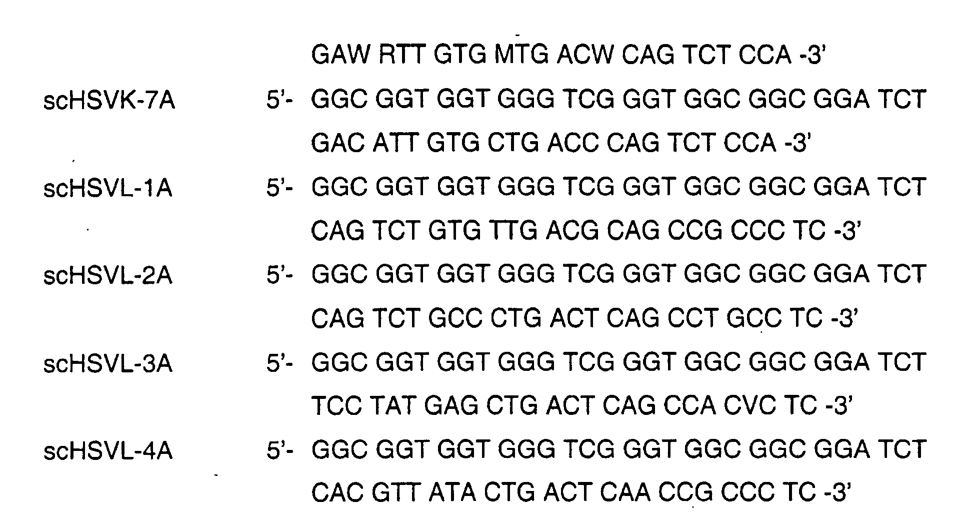

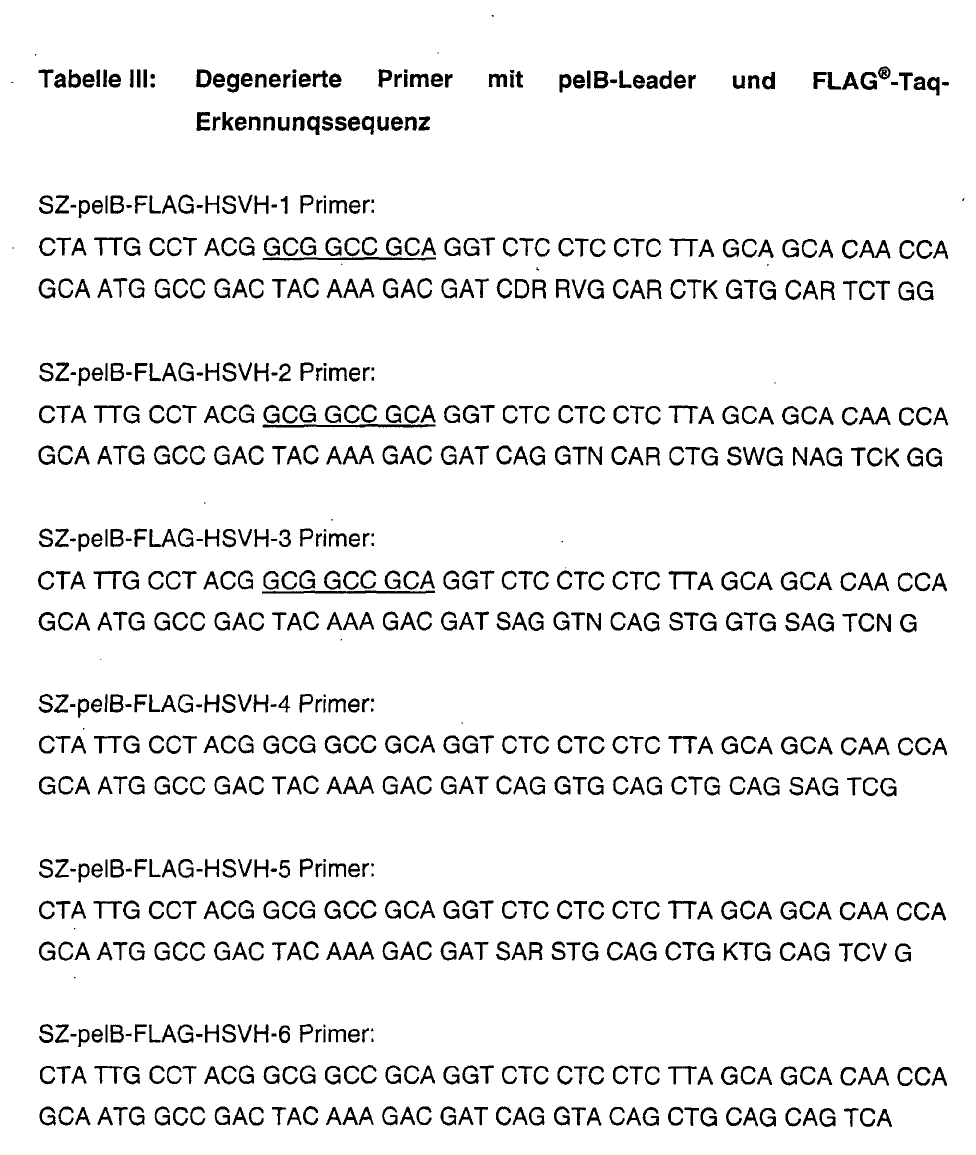

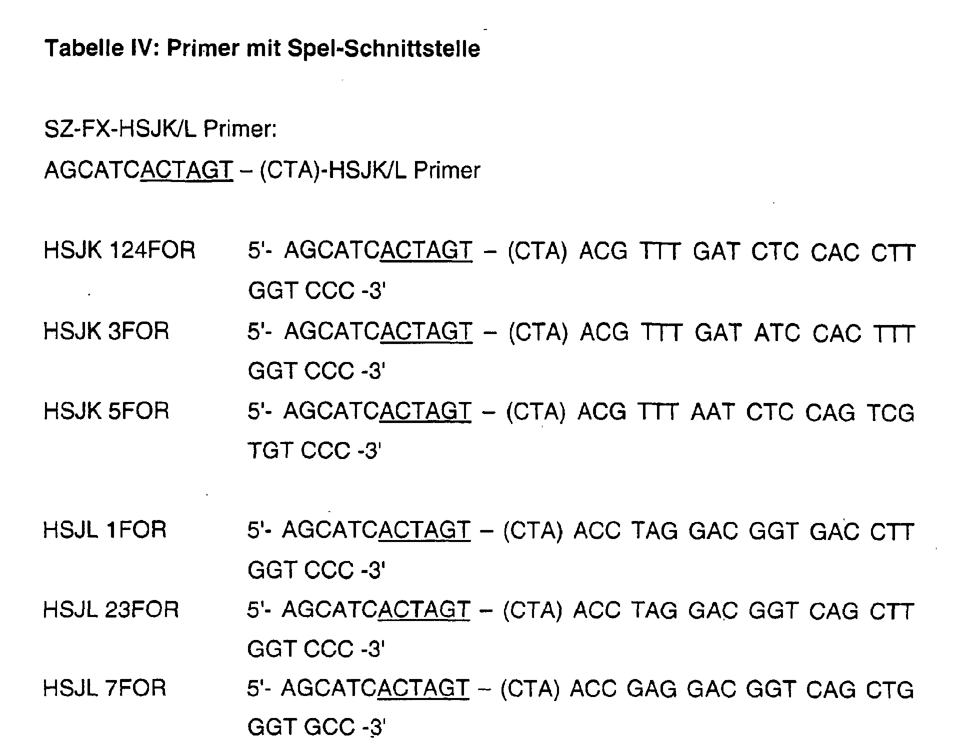

## Patentansprüche

1. Verwendung von dendritischen Zellen, die in vitro beladen wurden mit einem Bacteriophagen oder einem funktionell äquivalentem Fragment davon, der/das mindestens ein tumorspezifisches und/oder tumorassoziiertes Antigen mit einer Mindestlänge von 20 Aminosäuren als Fusionsprotein mit einem Phagenhüllprotein cpVIII oder einem Fragment oder einem funktionell äquivalenten Derivat davon auf seiner Oberfläche exprimiert zur Herstellung eines Vakzins zur Behandlung von Tumoren in Vertebraten.

2. Verwendung eines Bacteriophagen oder eines funktionell äquivalenten Fragmentes davon, der/das mindestens ein tumorspezifisches und/oder tumorassoziiertes Antigen mit einer Mindestlänge von 20 Aminosäuren als Fusionsprotein mit einem Phagenhüllprotein cpVIII oder einem Fragment oder einem funktionell äquivalenten Derivat davon auf seiner Oberfläche exprimiert, zur Beladung von dendritischen Zellen zur Herstellung eines Vakzins zur Behandlung von Tumoren in Vertebraten.

3. Verwendung nach Anspruch 1 oder 2, wobei der Phage oder das funktionell äquivalente Fragment davon mindestens ein Cytokin als Fusionsprotein mit dem Phagenhüllprotein oder dem funktionell äquivalenten Derivat davon auf seiner Oberfläche koexprimiert.

4. Verwendung nach einem der Ansprüche 1 bis 3, wobei der Bacteriophage ein filamentöser Phage ist.

5. Verwendung nach Anspruch 4, wobei der filamentöse Phage ein Klasse I-Phage, vorzugsweise ein fd-, M13-, f1-, If1-, Ike-, ZJ/Z- oder Ff-Phage ist oder ein Klasse II-Phage, vorzugsweise ein Xf-, Pf1- oder Pf3-Phage ist.

6. Verwendung nach einem der Ansprüche 1 bis 5, wobei das mindestens eine tumorspezifische und/oder tumorassoziierte Antigen als Fusionsprotein mit cpVIII oder einem funktionell äquivalenten Fragment davon und das mindestens eine Cytokin als Fusionsprotein mit cpIII oder einem funktionell äquivalenten Fragment davon exprimiert wird.

7. Verwendung nach einem der Ansprüche 1 bis 6, wobei das mindestens eine tumorspezifische und/oder tumorassoziierte Antigen ein Einzelstrang-fragment eines lymphomspezifischen Idiotypproteins oder eine antigene Determinante davon ist.

8. Verwendung nach einem der Ansprüche 1 bis 7, wobei der Tumor/die Neoplasie eine Hämoblastose, vorzugsweise ein malignes Lymphom oder eine Leukämie, ein malignes Melanom, ein urogenitales Karzinom, vorzugsweise ein Nieren-, Blasen-, Prostata- oder Hodenkarzinom, ein gynäkologisches Karzinom, vorzugsweise ein Mamma-, Ovarial-, Uterus- oder Zervixkarzinom, ein gastrointestinales Karzinom, vorzugsweise ein Ösophagus-, Magen-, Kolon-, Rektum-, Pankreas-, Gallenblasen-, Gallengangs- oder hepatocelluläres Karzinom, ein bösartiger endokrinologischer Tumor, vorzugsweise ein Schilddrüsenkarzinom, ein maligner Lungentumor, vorzugsweise ein Bronchialkarzinom oder Mesotheliom, ein Sarkom oder ein ZNS-Tumor ist.

9. Verwendung nach einem der Ansprüche 1 bis 8, wobei der Vertebrat ein Säuger, vorzugsweise ein Mensch, ist.
